# EUROPEAN PATENT SPECIFICATION

(11) **EP 1 395 279 B1**
(45) Date of publication and mention of the grant of the patent: **05.10.2011**
(21) Application number: 02719102.2
(22) Date of filing: 28.02.2002
(51) Int. Cl.: A61K 38/18

(54) **USE OF NEUBLASTIN POLYPEPTIDES FOR TREATING NEUROPATHIC PAIN**
VERWENDUNG VON NEUBLASTIN-POLYPEPTIDE ZUR BEHANDLUNG VON NEUROPATHISCHEN SCHMERZEN
TRAITEMENT UTILISANT DES POLYPEPTIDES DE NEUBLASTINE

(30) Priority: 28.03.2001 US 287554 P; 28.03.2001 US 820421
(43) Date of publication of application: 10.03.2004
(73) Proprietor: Biogen Idec MA Inc., Cambridge, Massachusetts 02142 (US)
(72) Inventor: SAH, Dinah, W., Y., Boston, MA 02114 (US)
(74) Representative: Pohlman, Sandra M.
(86) International application number: PCT/US2002/006388
(87) International publication number: WO 2002/078730

(56) References cited:
- WO-A-00/01815
- WO-A-00/04050
- WO-A-00/18799
- WO-A-00/34475
- WO-A-02/051433
- WO-A-02/060929
- WO-A-02/072826
- BOUCHER T J ET AL: "ARTEMIN PREVENTS INJURY-INDUCED CHANGES IN SMALL SENSORY NEURONS" ABSTRACTS OF THE SOCIETY FOR NEUROSCIENCE, SOCIETY FOR NEUROSCIENCE, WASHINGTON, DC, US, vol. 26, no. 1/2, 4 November 2000 (2000-11-04), page 63305 XP001121845 ISSN: 0190-5295

## Description

### FIELD OF THE INVENTION

The invention relates to uses of a neublastin polypeptide for use in treatments for neuropathic pain, wherein the neuropathic pain is allodynia, hyperalgesic pain or phantom pain.

### BACKGROUND OF THE INVENTION

Neuropathic pain is a category of pain that includes several forms of chronic pain and which results from dysfunction of nervous rather than somatic tissue. Neuropathic pain, that is pain deriving from dysfunction of the central or peripheral nervous system, may also be a consequence of damage to peripheral nerves or to regions of the central nervous system, may result from disease, or may be idiopathic. Symptoms of neuropathic pain include sensations of burning, tingling, electricity, pins and needles, stiffness, numbness in the extremities, feelings of bodily distortion, allodynia (pain evoked by stimulation of the skin that is normally innocuous), hyperalgesia (abnormal sensitivity to pain), and hyperpathia (an exaggerated pain response persisting long after the pain stimuli cease).

Several common causes of neuropathic pain are diabetes, cancer chemotherapy, herpes zoster infection, cervical or lumbar root compression owing to degenerative spine disease, malignant lesions of nerve plexus or root, nerve degeneration, such as from amputation, HIV infection, and lesions of central pain pathways, including the spinothalamic tract, thalamus, or thalamic radiations. Additional causes of neuropathic pain include drug-induced, or toxin-induced neuropathies. For example, antivirals such as ddI, ddC and d4T commonly cause peripheral neuropathies, as do phenytoin (a seizure medication), isoniazid (a tuberculosis medication), vincristine, vinblastine, taxol, taxotere and cisplatin (cancer chemotherapeutic agents), high dose vitamins, and folic acid antagonists.

Current therapies for the management of neuropathic pain are of limited benefit to many patients, and involve undesirable side effects or dose-limiting toxicities. In addition, current therapies are symptomatic, not disease modifying. Needs remain for improved therapies for the management and treatment of neuropathic pain, especially those that target the underlying pathology.

### SUMMARY OF THE INVENTION

This invention provides improved uses of a neublastin polypeptide for use in treating, preventing or delaying neuropathic pain, wherein the neuropathic pain is allodynia, hyperalgesic pain or phantom pain. The present uses apply neublastin ("NBN") polypeptides, including full-length neublastin polypeptides or bioactive truncated neublastin polypeptides, including, *e.g.*, at least SEQ ID NOS:2, 4, 5 and 11-27. In addition, the invention provides pharmaceutical compositions containing a full-length neublastin polypeptide or a truncated neublastin polypeptide suspended, dissolved, or dispersed in a pharmaceutically acceptable carrier for use in the treatment, prevention or delay of neuropathic pain, wherein the neuropathic is allodynia, hyperalgesic pain or phantom pain.

In a specific embodiment, the neublastin polypeptide may be any polypeptide of AA₋₈₀-AA₁₄₀ of SEQ ID NO:2, AA₋₄₁-AA₁₄₀ of SEQ ID NO:2, AA₁-AA₁₄₀ of SEQ ID NO:2, AA₂₅-AA₁₄₀ of SEQ ID NO:2, AA₂₈-AA₁₄₀ of SEQ ID NO:2, AA₈₀-AA₁₄₄ of SEQ ID NO:4, AA₁-AA₁₄₄ of SEQ ID NO:4, AA₁-AA₂₂₄ of SEQ ID NO:5, or AA₈₁-AA₂₂₄ of SEQ ID NO:5; at least one polypeptide comprising the C-terminal sequence set forth in either AA₁₀₇-AA₁₄₀ of SEQ ID NO:2 or AA₇₆-AA₁₄₀ of SEQ ID NO:2, and which retain the seven Cys residues characteristic of the GDNF family and of the TGF-beta super family; at least one polypeptide comprising SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 or SEQ ID NO:27; or at least one polypeptide sequence that has greater than 70% amino acid homology to a sequence listed above.

The neublastin polypeptide may be modified by a derivative moiety to have an extended residence time and or increased concentration in the body. The neublastin polypeptides may be N-glycosylated neublastin polypeptides. In addition, the neublastin polypeptide may be derivatized with one or more moieties including, but not limited to, polyethylene glycol moieties, aliphatic esters, amides, N-acyl-derivatives, or O-acyl derivatives.

In one embodiment, the invention features a use of a neublastin polypeptide including, *e.g.*, any one of SEQ ID NOS:2, 4, 5 and 11-27, for use in treating, preventing or delaying neuropathic pain, wherein the neuropathic pain is allodynia, hyperalgesic pain or phantom pain in a subject, either alone, or in combination with an analgesia-inducing compound selected from the group consisting of opioids, anti-arrhythmics, topical analgesics, local anaesthetics, anticonvulsants, antidepressants, corticosteroids and non-steroidal anti-inflammatory drugs (NSAIDS). In a preferred embodiment, the analgesia-inducing compound is an anticonvulsant. In another preferred embodiment, the analgesia-inducing compound is gabapentin ((1-aminomethyl)cyclohexane acetic acid) or pregabalin (S-(+)-4-amino-3-(2-methylpropyl) butanoic acid).

In another embodiment, the invention features a use of a neublastin polypeptide, including, *e.g.*, at least one of SEQ ID NOS:2, 4, 5 and 11-27 for treating tactile allodynia in a subject either alone, or in combination with an analgesia-inducing compound selected from the group consisting of opioids, anti-arrhythmics, topical analgesics, local anaesthetics, anticonvulsants, antidepressants, corticosteroids and NSAIDS. In a preferred embodiment, the analgesia-inducing compound is an anticonvulsant. In another preferred embodiment, the analgesia-inducing compound is gabapentin ((1-aminomethyl)cyclohexane acetic acid) or pregabalin (S-(+)-4-amino-3-(2-methylpropyl)butanoic acid).

Neublastin polypeptide may be administered in association with a therapeutic agent, including but not limited to an anti-cancer agent or an anti-viral agent. Anti-cancer agents include, but are not limited to, taxol, taxotere, cisplatin, nocodazole, vincristine, vindesine and vinblastine. Anti-viral agents include, but are not limited to, ddI, DDC, d4T, foscarnet, dapsone, metronidazole, and isoniazid.

The invention includes a use of a neublastin polypeptide for use in treating, preventing or delaying neuropathic pain in a subject, wherein the neuropathic pain is allodynia, hyperalgesic pain or phantom pain. In a specific embodiment, the neuropathic pain is associated with diabetic neuropathy. In another embodiment, the neuropathic pain is associated with infection of a subject by a virus, including but not limited to a herpes virus, a human immunodeficiency virus (HIV), and a papilloma virus. Neuropathic pain may be associated with infection by a herpes zoster virus, or especially with post-herpetic neuralgia. In a further embodiment, the neuropathic pain is associated with sciatica. Also described is a use for modulating the loss of pain sensitivity in a subject afflicted with a neuropathy, such as diabetic neuropathy. The loss of pain sensitivity may be a loss in thermal pain sensitivity.

In further embodiments, the neuropathic pain is hyperalgesic pain, phantom pain, or thermal hyperalgesia. In addition, neuropathic pain may also be associated with hereditary neuropathy (including but not limited to Friedreich ataxia, familial amyloid polyneuropathy, Tangier disease, Fabry disease), metabolic disorders (including but not limited to renal insufficiency and hypothyroidism), vitamin deficiencies (including but not limited to vitamin B12 deficiency, vitamin B6 deficiency, and vitamin E deficiency), toxic and iatrogenic neuropathies (including but not limited to alcoholism, vitamin B6 intoxication, hexacarbon intoxication, amiodarone, chloramphenicol, disulfiram, isoniazide, gold, lithium, metronidazole, misonidazole, nitrofurantoin), infectious neuropathies (including but not limited to leprosy, Lyme disease), auto-immune neuropathies (including but not limited to Guillain-Barre syndrome, chronic inflammatory de-myelinating polyneuropathy, monoclonal gammopathy of undetermined significance and polyneuropathy), trigeminal neuralgia, entrapment syndromes (including but not limited to Carpel tunnel), post-traumatic neuralgia, phantom limb pain, multiple sclerosis pain, complex regional pain syndromes (including but not limited to reflex sympathetic dystrophy, causalgia), neoplasia, vasculitic/angiopathic neuropathy and idiopathic neuropathy.

The foregoing uses contemplate administering to the subject, preferably systemically, a formulation comprising a neublastin polypeptide at a dosage of between 1 µg/kg to 30,000 µg/kg body weight of the subject, per dose. In alternative embodiments, the dosage is between 10 µg/kg to 30,000 µg/kg body weight of the subject, per dose; between 10 µg/kg to 10,000 µg/kg body weight of the subject, per dose; between 25 µg/kg to 10,000 µg/kg body weight of the subject, per dose; between 25 µg/kg to 3,000 µg/kg body weight of the subject, per dose; and between 50 µg/kg to 3,000 µg/kg body weight of the subject, per dose.

The neublastin polypeptide applied in the foregoing uses can be administered via any suitable delivery system, and preferably from the group consisting of intravenous delivery, intramuscular delivery, intrapulmonary delivery, subcutaneous delivery, and intraperitoneal delivery, most preferably via intramuscular delivery or subcutaneous delivery. The neublastin polypeptide applied in the foregoing uses can also be administered via intrathecal delivery.

The NBN polypeptide-containing formulation of the invention may be administered in a timed-released composition.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. In the case of conflict, the present specification,

including definitions, will control. In addition, the materials, methods, and examples are illustrative only and not intended to be limiting.

Other features and advantages of the invention will be apparent from the following detailed description and claims.

### BRIEF DESCRIPTION OF THE DRAWINGS

FIG. 1 is a broken line plot illustrating near complete prevention of tactile allodynia by subcutaneous neublastin (NBN) in rats with L5/L6 spinal nerve ligation (SNL);
FIG. 2 is a broken line plot illustrating near complete prevention of thermal hyperalgesia by subcutaneous neublastin (NBN) in rats with L5/L6 spinal nerve ligation (SNL).
FIG. 3 is a broken line plot illustrating the near complete reversal of fully established tactile allodynia by subcutaneous neublastin (NBN) in rats with L5/L6 spinal nerve ligation (SNL);
FIG. 4 is a broken line plot illustrating the near complete reversal of fully established thermal hyperalgesia by subcutaneous neublastin (NBN) in rats with L5/L6 spinal nerve ligation (SNL).
FIG. 5 is a bar graph illustrating near complete normalization of thermal hypoalgesia by subcutaneous neublastin in rats with STZ (streptozotocin) -induced neuropathy.
FIG. 6A and FIG. 6B are graphic representations illustrating the prevention of thermal hyperalgesia (FIG. 6A), prevention of thermal hypoalgesia (FIG. 6B), and reversal of thermal hyperalgesia (FIG. 6B) by subcutaneous neublastin in rats with STZ (streptozotocin) - induced neuropathy at 4 weeks (FIG. 6A) and 8 weeks (FIG. 6B) post STZ treatment.
FIG. 7 is a broken line plot illustrating dose-dependent neublastin (NBN) reversal of fully established tactile allodynia by subcutaneous neublastin in rats with L5/L6 spinal nerve ligation (SNL).
FIG. 8 is a broken line plot illustrating dose-dependent neublastin (NBN) reversal of fully established thermal hyperalgesia by subcutaneous neublastin in rats with L5/L6 spinal nerve ligation (SNL).

### DETAILED DISCLOSURE OF THE INVENTION

This invention relates to uses of a neublastin polypeptide and compositions comprising the same for treating, preventing or delaying neuropathic pain, wherein the neuropathic pain allodynia, hyperalgesic pain or phantom pain in a subject at risk of, or afflicted with, said neuropathic pain.

Neublastin polypeptides are proteins which promote survival, maintain phenotypic differentiation, prevent degeneration, promote regeneration, and restore the activity of neuronal cells and tissues. Neublastin (initially described, *e.g*., in WO 00/01815) has alternately been referred to as "artemin" (see, *e.g.,* WO 00/18799) and "enovin" (see, *e.g.*, WO 00/04050). Neublastin has been classified as a distant member of the TGF-β superfamily (Massague, et al,. 1994, Trends in Cell Biology, 4: 172-178) and is a member of glial cell line-derived neurotrophic factor ligand family ("GDNF"; WO 93/06116), in the family which includes GDNF, persephin ("PSP" ; Milbrandt et al., 1998, Neuron 20:245-253) and neurturin ("NTN"; WO 97/08196). The ligands of the GDNF subfamily have in common their ability to induce signalling through the RET receptor tyrosine kinase. These three ligands of the GDNF subfamily differ in their relative affinities for a family of neurotrophic receptors, the GFRα receptors. Neublastin acts preferably through the GFRα3 - RET complex. Baudet et al., Development, 127, pp. 4335-44 (2000); Baloh et al., Neuron, 21, pp. 1291-1302 (1998); Airaksinen et al., Mol. Cell. Neuroscience, 13, pp. 313-325 (1999).

An amino acid sequence comparison of Neublastin (SEQ ID NO:2) to the GDNF subfamily members Neurturin (SEQ ID NO:6), Persephin (SEQ ID NO:7) and GDNF (SEQ ID NO:8) is shown in Table 1. Neublastin polypeptides useful in this invention preferably hold the GDNF subfamily fingerprint, *i.e.* the amino acid residues underlined in Table 1.

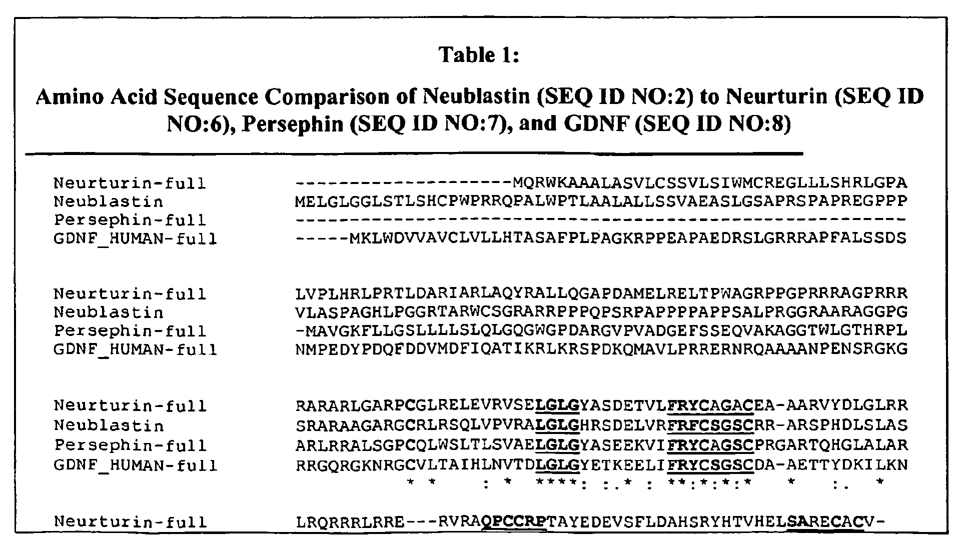

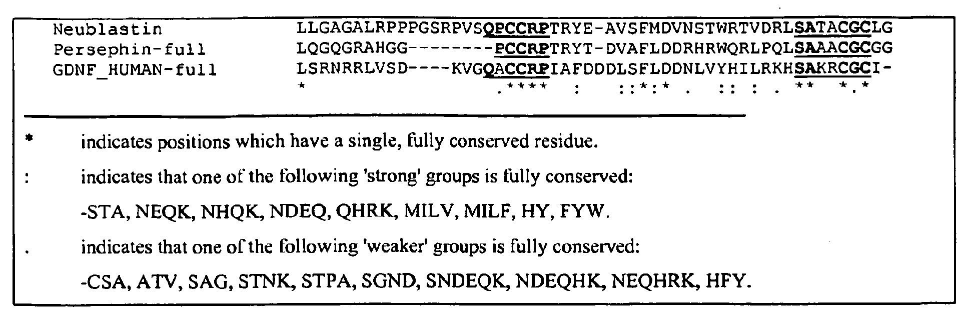

From the amino acid sequence alignment shown in Table 1, it can be seen that neublastin has seven cysteine residues at locations that are conserved within the TGF-β superfamily. Based on this sequence alignment, neublastin was shown to be a member of the GDNF subfamily of neurotrophic factors (LGLG - FR(Y/F)CSGSC - QxCCRP - SAxxCGC, the GDNF subfamily fingerprint, underlined in Table 1).

The neublastin polypeptides useful herein may be provided in any bioactive form, including the form of pre-pro-proteins, pro-proteins, mature proteins, glycosylated proteins, phosphorylated proteins, truncated forms, or any other post-translationally modified protein. It is assumed that a bioactive neublastin is in the dimerized form for each NBN variant, because dimer formation is required for activity. Little to no activity is observed in a monomeric NBN polypeptide. A bioactive neublastin polypeptide includes a dimerized polypeptide that, in the presence of a cofactor (such as GFRα3 or RET), binds to GFRα3 or to a complex of GFRα3 and RET, induces dimerization of RET, and autophosphorylation of RET. Accordingly, a "neublastin polypeptide," as used herein, is a polypeptide which possesses neurotrophic activity (*e.g.*, as described in WO 00/01815) as follows:

### 1. Wild-type Neublastin

The following "wild-type" neublastin amino acid ("aa" or "AA") sequences are exemplary of those that are applied in the uses of the compositions of this invention:
-- AA₈₀-AA₁₄₀ of SEQ ID NO:2 ("wild type" human prepro),
-- AA₋₄₁-AA₁₄₀ of SEQ ID NO:2 (pro human),
-- AA₁-AA₁₄₀ of SEQ ID NO:2 (mature 140AA (SEQ ID NO: 11); hereafter "140NBN"),
-- AA₂₅-AA₁₄₀ of SEQ ID NO:2 (mature 116AA (SEQ ID NO:12); hereafter "116NBN"),
-- AA₂₈-AA₁₄₀ of SEQ ID NO:2 (mature 113AA (SEQ ID NO:13); hereafter "113NBN"),
-- AA₋₈₀-AA₁₄₄ of SEQ ID NO:4 (murine prepro),
-- AA₁-AA₁₄₄ of SEQ ID NO:4 (murine mature -- 144 AA),
-- AA₁-AA₂₂₄ of SEQ ID NO:5 (rat prepro),
-- AA₈₁-AA₂₂₄ of SEQ ID NO:5 (rat mature -- 144 AA),
-- Peptides with a C-terminal sequence set forth in AA₁₀₇-AA₁₄₀ of SEQ ID NO:2, more preferably AA₇₆-AA₁₄₀ of SEQ ID NO:2, and which retain the 7 Cys residues characteristic of the GDNF family and of the TGF-β super family.

In one embodiment, the preferred neublastin polypeptide contains (seven) cysteines conserved as in SEQ ID NO:2 at positions 43, 70, 74, 107, 108, 136 and 138. These seven conserved cysteine residues are known within the TGF- β superfamily to form three intramonomeric disulfide bonds (contemplated, *e.g*., in SEQ ID NO:2 between cysteine residues 43-108, 70-136, and 74-138) and one intermonomeric disulfide bond (contemplated, *e.g*., in SEQ ID NO:2 between cysteine residues 107-107), which together with the extended beta strand region constitutes the conserved structural motif for the TGF- β superfamily. See, *e.g.*, Daopin et al., Proteins 1993, 17: 176-192.

### 2. Truncated Neublastins ("NBNs")

Neublastin polypeptides useful in the present invention also include truncated forms of the full length neublastin molecule. In such truncated molecules, one or more amino acids have been deleted from the N-terminus or the C-terminus, preferably the N-terminus. The truncated neublastin polypeptide may be obtained by providing a mature neublastin polypeptide and contacting the mature neublastin polypeptide with at least one protease under conditions sufficient to produce the truncated neublastin polypeptide. Preferably, at least one protease is an exoprotease, and contacting the mature neublastin polypeptide results in formation of an exopeptidase neublastin polypeptide digestion product that can be further digested with a dipeptidyl peptidase.

The truncated neublastin polypeptides described herein preferably include a polypeptide sequence that encompasses the seven cysteine residues conserved in the mature neublastin sequence. In certain preferred embodiments, the truncated neublastin polypeptide includes at least the 85 carboxy terminal amino acids of mature 113NBN neublastin polypeptide.

Other variants of Neublastin include truncated NBN forms. Examples of these include:
*(i)* the 112AA polypeptide sequence designated herein as NBN112, which possesses the carboxy terminal 112 amino acids of a mature neublastin polypeptide, *e.g.*, amino acids 29-140 of SEQ ID NO:2 (SEQ ID NO:14).
*(ii)* the 111AA polypeptide sequence designated herein as NBN111, which possesses the carboxy terminal 111 amino acids of a mature neublastin polypeptide, *e.g.*, amino acids 30-140 of SEQ ID NO:2 (SEQ ID NO:15).
*(iii)* the 110AA polypeptide sequence designated herein as NBN110, which possesses the carboxy terminal 110 amino acids of a mature neublastin polypeptide, *e.g.*, amino acids 31-140 of SEQ ID NO:2 (SEQ ID NO:16).
*(iv)* the 109AA polypeptide sequence designated herein as NBN109, which possesses the carboxy terminal 109 amino acids of a mature neublastin polypeptide, *e.g.*, amino acids 32-140 of SEQ ID NO:2 (SEQ ID NO:17).
*(v)* the 108AA polypeptide sequence designated herein as NBN108, which possesses the carboxy terminal 108 amino acids of a mature neublastin polypeptide, *e.g*., amino acids 33-140 of SEQ ID NO:2 (SEQ ID NO:18).
*(vi)* the 107AA polypeptide sequence designated herein as NBN107, which possesses the carboxy terminal 107 amino acids of a mature neublastin polypeptide, *e.g.*, amino acids 34-140 of SEQ ID NO:2 (SEQ ID NO:19).
*(vii)* the 106AA polypeptide sequence designated herein as NBN106, which possesses the carboxy terminal 106 amino acids of a mature neublastin polypeptide, *e.g.*, amino acids 35-140 of SEQ ID NO:2 (SEQ ID NO:20).
*(viii)* the 105AA polypeptide sequence designated herein as NBN105, which possesses the carboxy terminal 105 amino acids of a mature neublastin polypeptide, *e.g.*, amino acids 36-140 of SEQ ID NO:2 (SEQ ID NO:21).
*(ix)* the 104AA polypeptide sequence designated herein as NBN104, which possesses the carboxy terminal 104 amino acids of a mature neublastin polypeptide, *e.g*., amino acids 37-140 of SEQ ID NO:2 (SEQ ID NO:22).
*(x)* the 103AA polypeptide sequence designated herein as NBN103, which possesses the carboxy terminal 103 amino acids of a mature neublastin polypeptide, *e.g.*, amino acids 38-140 of SEQ ID NO:2 (SEQ ID NO:23).
*(xi)* the 102AA polypeptide sequence designated herein as NBN102, which possesses the carboxy terminal 102 amino acids of a mature neublastin polypeptide, *e.g*., amino acids 39-140 of SEQ ID NO:2 (SEQ ID NO:24).
*(xii)* the 101AA polypeptide sequence designated herein as NBN101, which possesses the carboxy terminal 101 amino acids of a mature neublastin polypeptide, *e.g*., amino acids 40-140 of SEQ ID NO:2 (SEQ ID NO:25).
*(xiii)* the 100AA polypeptide sequence designated herein as NBN100, which possesses the carboxy terminal 100 amino acids of a mature neublastin polypeptide, *e.g*., amino acids 41-140 of SEQ ID NO:2 (SEQ ID NO:26).
*(xiv)* the 99AA polypeptide sequence designated herein as NBN99, which possesses the carboxy terminal 99 amino acids of a mature neublastin polypeptide, *e.g*., amino acids 42-140 of SEQ ID NO:2 (SEQ ID NO:27).

It is understood that the truncated forms of Neublastin disclosed herein (*e.g*., the 112AA through 99AA forms) have neurotrophic activity.

In most preferred embodiments, the truncated neublastin polypeptide is the 99 aa, 100 aa, 101 aa, 102 aa, 103 aa, 104 aa, 105 aa, 106 aa, 107 aa, 108 aa, 109 aa, 110 aa, 111 aa or 112 aa carboxy terminal amino acids of mature 113 AA neublastin polypeptide (*i.e.*, NBN99, NBN100, NBN101, NBN102, NBN103, NBN104, NBN105, NBN106, NBN107, NBN108, NBN 109, NBN110, NBN111 or NBN112, respectively). The sequences may also be found in the murine and rat neublastin polypeptides as the carboxy terminal 99 aa, 100 aa, 101 aa, 102 aa, 103 aa, 104 aa, 105 aa, 106 aa, 107 aa, 108 aa, 109 aa, 110 aa, 111 aa or 112 aa, respectively, in SEQ ID NOS:4 and 5. These most preferred examples of truncated NBN forms are bioactive (referred to "bioactive truncated neublastin polypeptides") as they have been demonstrated herein to have neurotrophic activity. As stated above, NBN dimerization is required for bioactivity, as little to no activity is observed with the NBN monomeric polypeptide.

### 3. Variant Neublastins ("NBNs") With Substantial Similarity or Identity

The NBNs useful in this invention also include those NBN polypeptides that have an amino acid sequence with substantial similarity or identity to the various prepro, pro, mature and truncated "neublastin" polypeptides set forth above. Preferably the neublastin polypeptide used has at least 70%, more preferably 85%, still more preferably 90%, or still further preferably 95% identity or similarity to the neublastin polypeptides in SEQ ID NOS:2, 4, 5 or 11-27. Most preferably the neublastin polypeptide used has at least 99% similarity or identity to the neublastin polypeptides in SEQ ID NOS:2, 4, 5 or 11-27.

The degree to which a candidate polypeptide shares homology with a neublastin polypeptide of the invention is determined as the degree of similarity or identity between two amino acid sequences.

A high level of sequence identity indicates a likelihood that the first sequence is derived from the second sequence. Amino acid sequence identity requires identical amino acid sequences between two aligned sequences. Thus, a candidate sequence sharing 70% amino acid identity with a reference sequence, requires that, following alignment, 70% of the amino acids in the candidate sequence are identical to the corresponding amino acids in the reference sequence. Identity is determined by computer analysis, such as, without limitations, the ClustalX computer alignment program (Thompson JD, Gibson TJ, Plewniak F, Jeanmougin F, & Higgins DG: "The ClustalX windows interface: flexible strategies for multiple sequence alignment aided by quality analysis tools"; Nucleic Acids Res. 1997, 25 (24): 4876-82), and the default parameters suggested therein. Using this program, the mature part of a polypeptide encoded by an analogous DNA sequence of the invention exhibits a degree of identity of at least 70%, more preferably 85%, still more preferably 90%, or still further preferably 95%, most preferably at least 99% with the amino acid sequences presented herein as SEQ ID NO:2 (human NBN), SEQ ID NOS:4 and 5 (rodent NBN), or SEQ ID NOS:11-27 (mature and truncated NBN).

Other alignment tools are known, such as the dynamic programming algorithm described in Needleman et al., J. Mol. Biol. 48: 443 (1970), and the Align Program, a commercial software package produced by DNAstar, Inc. Once the alignment between the candidate and reference sequence is made and refined, a percent homology score is calculated. The individual amino acids of each sequence are compared sequentially according to their similarity to each other.

Similarity factors include similar size, shape and electrical charge. One particularly preferred method of determining amino acid similarities is the PAM25O matrix described in Dayhoff et al., ATLAS OF PROTEIN SEQUENCE AND STRUCTURE 345-352 (1978 & Supp.). A similarity score is first calculated as the sum of the aligned pairwise amino acid similarity scores. Insertions and deletions are ignored for the purposes of percent homology and identity. Accordingly, gap penalties are not used in this calculation. The raw score is then normalized by dividing it by the geometric mean of the scores of the candidate compound and the seven cysteine skeleton of the neublastin polypeptides. The geometric mean is the square root of the product of these scores. The normalized raw score is the percent homology.

As noted above, the neublastin polypeptides of the invention include variant polypeptides. In the context of this invention, the term "variant polypeptide" includes a polypeptide (or protein) having an amino acid sequence that differs from the sequences presented as SEQ ID NO:2 (human NBN), or SEQ ID NOS:4 and 5 (rodent NBN), or SEQ ID NOS:11-27 (mature and truncated NBN), at one or more amino acid positions. Such variant polypeptides include the modified polypeptides described above, as well as conservative substitutions, splice variants, isoforms, homologues from other species, and polymorphisms.

As defined herein, the term "conservative substitutions" denotes the replacement of an amino acid residue by another, biologically similar, residue. Typically, biological similarity, as referred to above, reflects substitutions on the wild type sequence with conserved amino acids. For example, one would expect conservative amino acid substitutions to have little or no effect on the biological activity, particularly if they represent less than 10% of the total number of residues in the polypeptide or protein. Preferably, conservative amino acid substitutions represent changes in less than 5% of the polypeptide or protein, most preferably less than 2% of the polypeptide or protein. For example, when calculated in accordance, *e.g*., with human 113NBN, most preferred conservative substitutions would represent fewer than three amino acid substitutions in the wild type mature amino acid sequence. In a particularly preferred embodiment, there is a single amino acid substitution in the mature sequence, wherein both the substituted and replacement amino acid are non-cyclic.

Other examples of particularly conservative substitutions include the substitution of one hydrophobic residue for another, such as isoleucine, valine, leucine or methionine, or the substitution of one polar residue for another, such as the substitution of arginine for lysine, glutamic for aspartic acid, or glutamine for asparagine, and the like.

The term conservative substitution also includes the use of a substituted amino acid residue in place of an un-substituted parent amino acid residue provided that antibodies raised to the substituted polypeptide also immunoreact with the un-substituted polypeptide.

Modifications of this primary amino acid sequence may result in proteins which have substantially equivalent activity as compared to the unmodified counterpart polypeptide, and thus may be considered functional analogs of the parent proteins. Such modifications may be deliberate, *e.g.* as by site-directed mutagenesis, or they may occur spontaneously, and include splice variants, isoforms, homologues from other species, and polymorphisms. Such functional analogs are also contemplated according to the invention.

Moreover, modifications of the primary amino acid sequence may result in proteins which do not retain the biological activity of the parent protein, including dominant negative forms, etc. A dominant negative protein may interfere with the wild-type protein by binding to, or otherwise sequestering regulating agents, such as upstream or downstream components, that normally interact functionally with the polypeptide. Such dominant negative forms are also contemplated according to the invention.

### 4. Derivative or Modified NBNs

Polypeptides of the present invention also include chimeric polypeptides or cleavable fusion polypeptides in which another polypeptide is fused at the N-terminus or the C-terminus of the polypeptide or fragment thereof. A chimeric polypeptide may be produced by fusing a nucleic acid sequence (or a portion thereof) encoding another polypeptide to a nucleic acid sequence (or a portion thereof) of the present invention. Techniques for producing chimeric polypeptides are standard techniques. Such techniques usually require joining the sequences such that they are in the same reading frame, and expression of the fused polypeptide under the control of the same promoter(s) and terminator.

The neublastin polypeptides may be N-glycosylated polypeptides. In one embodiment, the Asn residue at position 122 of SEQ ID NO:2 is glycosylated.

This invention also contemplates neublastin fusion proteins, such as Ig-fusions, as described, *e.g.*, in United States patents 5,434,131; 5,565,335; 5,541,087; and 5,726,044, or preferably serum albumin fusions.

The neublastin polypeptides useful here include timed-release compositions and neublastin polypeptides modified with a derivative moiety (preferably a polyethylene glycol moiety) to have an extended residence time and/or increased concentration in body fluids. In addition, the neublastin polypeptide may be derivatized with a moiety selected from the group consisting of aliphatic esters, amides, N-acyl-derivatives, or O-acyl derivatives.

Table 2 below shows a ClustalW comparison between human (SEQ ID NO:2), mouse (SEQ ID NO:4) and rat (SEQ ID NO:5) prepro-neublastin. Mature NBN polypeptides NBN 140, NBN 116, NBN 113 and NBN99 are indicated by "*". The N-terminus of NBN112 through NBN100 are indicated by consecutive ":" symbols. Table 3 below shows distance comparison between wild type human, mouse and rat neublastin polypeptides (SEQ ID NOS:2, 4 and 5).

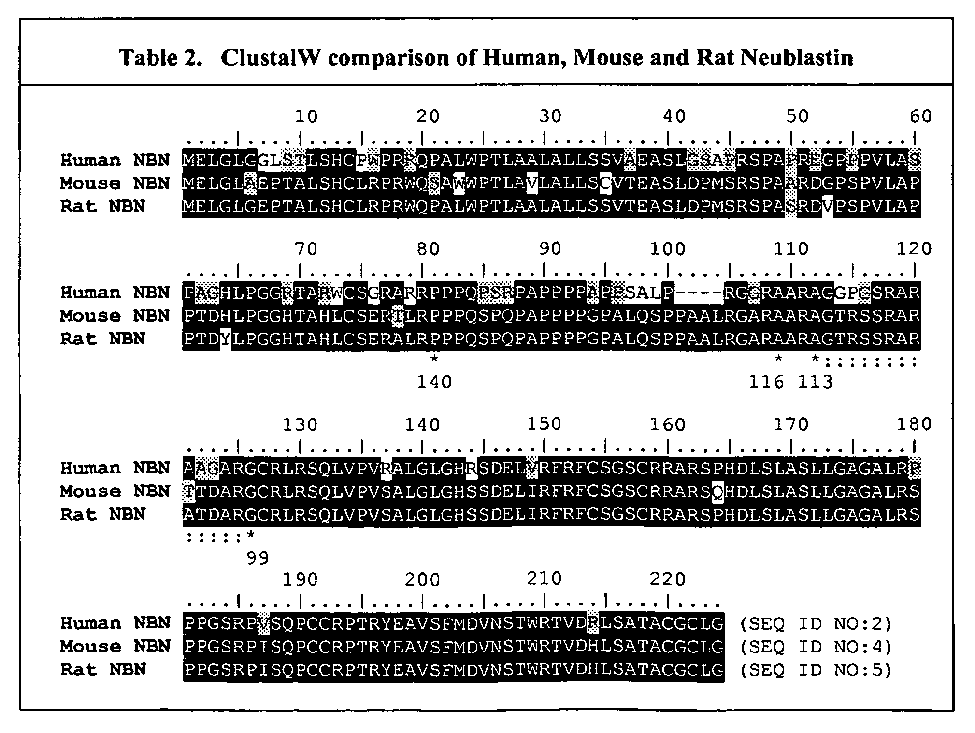

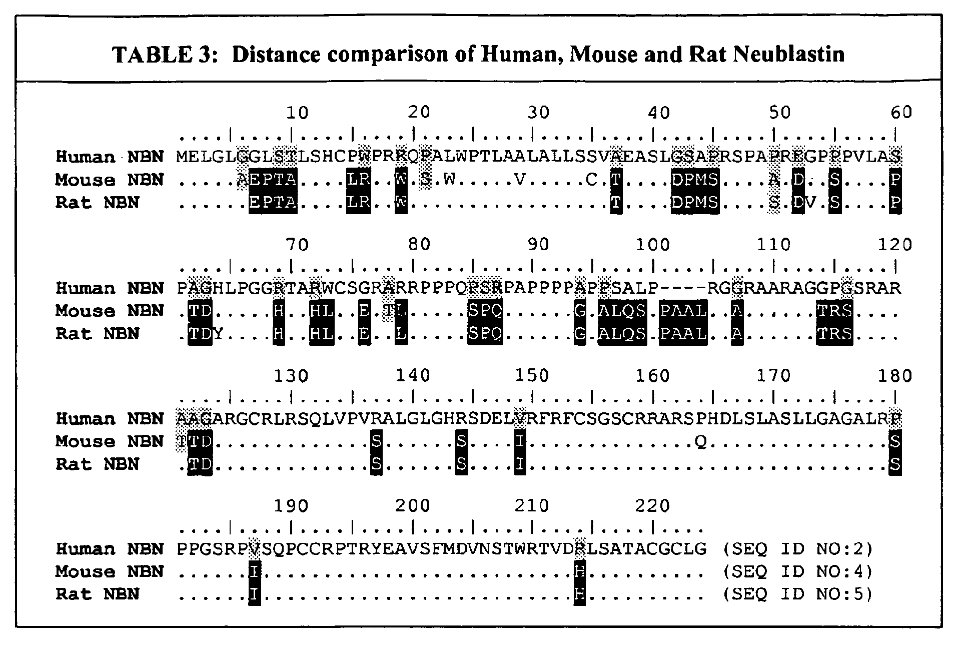

### Methods Of Producing The Neublastin Polypeptide

The neublastin polypeptide used herein may be isolated from mammalian cells, preferably from a human cell or from a cell of murine origin. In a most preferred embodiment, the neublastin polypeptide may be isolated from human heart tissue, from human skeletal muscle, from human pancreas, or from human brain tissue, in particular from caudate nucleus or from thalamus, or it may be obtained from DNA of mammalian origin, as discussed in more detail below.

Alternately, the neublastin polypeptides may be obtained by expression of polynucleotides that encode such neublastin polypeptides. Such polynucleotides include DNA, cDNA and RNA sequences and are available in the art. See, *e.g.*, WO 00/01815, WO 00/04050 and WO 00/18799. Particularly useful polynucleotides have the DNA sequence presented as SEQ ID NO:1 (human NBN cDNA), and the DNA sequence presented as SEQ ID NO:3 (mouse NBN cDNA). In addition, the genomic sequence for human NBN may be used (see GenBank Accession No. AC 005038).

More specifically, the neublastin polypeptides useful herein may be obtained by culturing a cell containing a nucleic acid sequence encoding a neublastin polypeptide under conditions permitting the production of the neublastin polypeptide, followed by recovery of the neublastin polypeptide from the culture medium. The nucleic acid sequence encoding a neublastin polypeptide may be a nucleic acid sequence that is normally endogenous to the cell or an exogenously-derived nucleic acid sequence that is introduced into a "production" cell. When cells are to be genetically modified for the purposes of producing a neublastin polypeptide, the cells may be modified by conventional methods or by gene activation.

According to conventional methods, a DNA molecule that contains a neublastin cDNA or genomic DNA sequence may be contained within an expression construct and transfected into cells by standard methods including, but not limited to, liposome-, polybrene-, or DEAE dextran-mediated transfection, electroporation, calcium phosphate precipitation, microinjection, or velocity driven microprojectiles ("biolistics"). Alternatively, one could use a system that delivers DNA by viral vector. Viruses known to be useful for gene transfer include adenoviruses, adeno-associated virus, lentivirus, herpes virus, mumps virus, poliovirus, retroviruses, Sindbis virus, and vaccinia virus such as canary pox virus, as well as Baculovirus infection of insect cells, in particular Sf9 insect cells.

Alternatively, the cells may be modified to produce a neublastin polypeptide using a gene activation ("GA") approach, such as described in United States patents 5,733,761 and 5,750,376.

Accordingly, the term "genetically modified," as used herein in reference to cells, is meant to encompass cells that express a particular gene product following introduction of a nucleic acid molecule encoding the gene product and/or regulatory elements that control expression of a endogenous coding sequence for the gene product. The nucleic molecule may be introduced by gene targeting, allowing incorporation of the nucleic molecule at a particular genomic site.

In one embodiment, the neublastin polypeptide is produced in a bacterial cell, preferably *E. coli*. In a different embodiment, the neublastin polypeptide is produced in an insect derived cell, particularly Sf9.

In another embodiment, the neublastin polypeptide is produced in, *e.g.*, a mammalian cell, *e.g**.***, a human cell, an oocyte, or a yeast cell. The cell of the invention may be without limitation a human embryonic kidney ("HEK") cell, *e.g.*, a HEK 293 cell, a BHK21 cell, a Chinese hamster ovary ("CHO") cell, a *Xenopus laevis* oocyte ("XLO") cell, or *Pichia pastoris* (yeast). In one embodiment, the cell of the invention is a fungal cell, *e.g.*, a filamentous fungal cell. In yet another embodiment, the cell is an insect cell, most preferably the Sf9 cell. Additional mammalian cells of the invention are PC12, HiB5, RN33b cell lines, human neural progenitor cells, and other cells derived from human cells, especially neural cells.

Examples of primary or secondary cells include fibroblasts, epithelial cells including mammary and intestinal epithelial cells, endothelial cells, formed elements of the blood including lymphocytes and bone marrow cells, glial cells, hepatocytes, keratinocytes, muscle cells, neural cells, or the precursors of these cell types. Examples of immortalized human cell lines useful in the present methods include, but are not limited to, Bowes Melanoma cells (ATCC Accession No. CRL 9607), Daudi cells (ATCC Accession No. CCL 213), HeLa cells and derivatives of HeLa cells (ATCC Accession Nos. CCL 2, CCL 2.1, and CCL 2.2), HL-60 cells (ATCC Accession No. CCL 240), HT-1080 cells (ATCC Accession No. CCL 121), Jurkat cells (ATCC Accession No. TIB 152), KB carcinoma cells (ATCC Accession No. CCL 17), K-562 leukemia cells (ATCC Accession No. CCL 243), MCF-7 breast cancer cells (ATCC Accession No. BTH 22), MOLT-4 cells (ATCC Accession No. 1582), Namalwa cells (ATCC Accession No. CRL 1432), Raji cells (ATCC Accession No. CCL 86), RPMI 8226 cells (ATCC Accession No. CCL 155), U-937 cells (ATCC Accession No. CRL 1593), WI-38VA13 sub line 2R4 cells (ATCC Accession No. CLL 75.1), and 2780AD ovarian carcinoma cells (Van der Blick et al., Cancer Res. 48: 5927-5932, 1988), as well as heterohybridoma cells produced by fusion of human cells and cells of another species. Secondary human fibroblast strains, such as WI-38 (ATCC Accession No. CCL 75) and MRC-5 (ATCC Accession No. CCL 171), may also be used.

When the cell is an eukaryotic cell, incorporation of the heterologous polynucleotide of the invention may in particular be carried out by infection (employing a virus vector), by transfection (employing a plasmid vector), using calcium phosphate precipitation, microinjection, electroporation, lipofection, or other physical-chemical methods known in the art.

The NBN polypeptides are isolated from production cell cultures, or from culture medium conditioned by production cells, using standard protein purification techniques including refolding if applicable. Suitable techniques are described below in the Examples.

### Subjects For Treatment

This invention can be used for the treatment, prophylaxis or delay of neuropathic pain, wherein the neuropathic pain is allodynia, hyperalgesic pain or phantom pain, in a mammalian subject afflicted therewith, or at risk thereof by applying a Neublastin polypeptide. Subjects at risk of developing a neuropathy include subjects with diabetes, subjects who are receiving chemotherapy, subjects who have experienced certain traumas, subjects who have ingested various toxins or drugs, subjects experiencing certain vitamin deficiencies, subjects infected with certain viral pathogens, subjects afflicted with various autoimmune disorders and metabolic disorders, and subjects who have experienced certain nerve damage or neurodegeneration. Mammalian subjects include sheep, horses, dogs, cats, pigs, rabbits, guinea pigs, rats, hamsters, gerbils and mice, but most preferably are humans.

Typically, in human subjects, the patient is either refractory to other traditional pain therapies, or the subject responds insufficiently to other such pain therapies to provide satisfactory pain control.

In general, this invention features both prophylactic treatment and therapeutic treatment protocols. In prophylactic treatment, a neublastin polypeptide is prepared to be administered to a subject at risk of neuropathic pain, wherein the neuropathic pain is allodynia, hyperalgesic pain or phantom pain; such subjects would be expected to be subjects with an early stage neuropathy. The treatment with neublastin under such circumstances would serve to treat at-risk patients preventively.

In therapeutic treatment, a neublastin polypeptide is prepared to be administered to a subject who has experienced neuropathic pain, wherein the neuropathic pain is allodynia, hyperalgesic pain or phantom pain; such subjects would be expected to be subjects with a late stage neuropathy. The treatment with neublastin under such circumstances would serve to alleviate said neuropathic pain. Such late stage patients may have received a number of therapies, beginning with self-medication (such as non-steroidal anti-inflammatory drugs, *e.g.*, ibuprofen). Such treatments may be escalated to antidepressants (*e.g.*, tricyclic antidepressants, venlafaxine, and selective serotonin re-uptake inhibitors -- specific medications include amitriptyline, desipramine, imipramine and nortriptyline), or anticonvulsants (*e.g.*, gabapentin, carbamazepine, lamotrigine, topiramate, and phenytoin). Other medication include topical analgesics (*e.g.*, capsaicin, and lidoderm), anti-arrhythmics and opioids. Surgery may be performed in severe neuropathy cases. Studies indicate that fewer than 50% of patients respond to topical analgesics, anti-arrhythmics, opioids or surgery.

### Methods and Pharmaceutical Compositions

This invention provides uses of a neublastin polypeptide for treating, preventing or delaying neuropathic pain, wherein the neuropathic pain is allodynia, hyperalgesic pain or phantom pain. The present uses apply neublastin polypeptides, including full length neublastin polypeptides or bioactive truncated neublastin polypeptides. In addition, the invention provides pharmaceutical compositions containing a full length neublastin polypeptide or a truncated neublastin polypeptide suspended, dissolved, or dispersed in a pharmaceutically acceptable carrier for treating, preventing or delaying neuropathic pain, wherein the neuropathic pain is allodynia, hyperalgesic pain or phantom pain.

### 1. Treatment of Neuropathic Pain

In one embodiment, the invention features a use of a neublastin polypeptide for treating, preventing or delaying neuropathic pain, wherein the neuropathic pain is allodynia, hyperalgesic pain or phantom pain. The neublastin polypeptide may be administered alone (mono-therapy) or as part of a combination therapy regime. Preferred combination therapies include an analgesia-inducing compound selected from the group consisting of opioids, anti-arrhythmics, topical analgesics, local anaesthetics, anticonvulsants, antidepressants, corticosteroids and non-steroidal anti-inflammatory drugs (NSAIDS).

The invention also provides treatments of chemotherapy-induced neuropathies (such as those caused by delivery of chemotherapeutic agents, *e.g.*, taxol or cisplatin); toxin-induced neuropathies, drug-induced neuropathies, pathogen-induced (*e.g.*, virus induced) neuropathies, vitamin-deficiency-induced neuropathies; idiopathic neuropathies; and diabetic neuropathies by applying a neublastin polypeptide. See, *e.g.*, United States Patent Nos. 5,496,804 and 5,916,555. The invention still further can be used for treatment of mono-neuropathies, mono-multiplex neuropathies, and poly-neuropathies, including axonal and demyelinating neuropathies, using the neublastin nucleotides and polypeptides of this invention.

The neuropathic pain may be associated with a number of peripheral neuropathies, including:
(a) chemotherapy-induced neuropathies,
(b) toxin-induced neuropathies (including but not limited to neuropathies induced by alcoholism, vitamin B6 intoxication, hexacarbon intoxication, amiodarone, chloramphenicol, disulfiram, isoniazide, gold, lithium, metronidazole, misonidazole, nitrofurantoin),
(c) drug-induced neuropathies, including therapeutic drug-induced neuropathic pain (such as caused by anti-cancer agents, particularly anti-cancer agents selected from the group consisting of taxol, taxotere, cisplatin, nocodazole, vincristine, vindesine and vinblastine; and such as caused by anti-viral agents, particularly anti-viral agents selected from the group consisting of ddI, DDC, d4T, foscarnet, dapsone, metronidazole, and isoniazid).
(d) vitamin-deficiency-induced neuropathies (including but not limited to vitamin B12 deficiency, vitamin B6 deficiency, and vitamin E deficiency),
(e) idiopathic neuropathies,
(f) diabetic neuropathies,
(g) pathogen-induced nerve damage,
(h) inflammation-induced nerve damage,
(i) neurodegeneration,
(j) hereditary neuropathy (including but not limited to Friedreich ataxia, familial amyloid polyneuropathy, Tangier disease, Fabry disease),
(k) metabolic disorders (including but not limited to renal insufficiency and hypothyroidism),
(l) infectious and viral neuropathies (including but not limited to neuropathic pain associated with leprosy, Lyme disease, neuropathic pain associated with infection by a virus, particularly a virus selected from the group consisting of a herpes virus (*e.g*. herpes zoster which may lead to post-herpetic neuralgia), a human immunodeficiency virus (HIV), and a papilloma virus),
(m) auto-immune neuropathies (including but not limited to Guillain-Barre syndrome, chronic inflammatory de-myelinating polyneuropathy, monoclonal gammopathy of undetermined significance and polyneuropathy),
(n) trigeminal neuralgia and entrapment syndromes (including but not limited to Carpel tunnel),
(o) other neuropathic pain syndromes including post-traumatic neuralgia, phantom limb pain, multiple sclerosis pain, complex regional pain syndromes (including but not limited to reflex sympathetic dystrophy, causalgia), neoplasia- associated pain, vasculitic/angiopathic neuropathy, and sciatica.

Neuropathic pain may be manifested as allodynia, hyperalgesic pain, thermal hyperalgesia, or phantom pain.

### 2. Treatment of Tactile Allodynia

The term "tactile allodynia" typically refers to the condition in a subject where pain is evoked by stimulation of the skin (*e.g.* touch) that is normally innocuous. This invention features a use of a neublastin polypeptide for use in treating tactile allodynia in a subject. In one embodiment, tactile allodynia is treated by a neublastin polypeptide alone.

In a second embodiment, tactile allodynia is treated by administering to the subject an effective amount of a neublastin polypeptide in combination with an effective amount of an analgesia-inducing compound selected from the group consisting of opioids, anti-arrhythmics, topical analgesics, local anaesthetics, anticonvulsants, antidepressants, corticosteroids and NSAIDS. In a preferred embodiment, the analgesia-inducing compound is an anticonvulsant. In another preferred embodiment, the analgesia-inducing compound is gabapentin ((1-aminomethyl)cyclohexane acetic acid) or pregabalin (S-(+)-4-amino-3-(2-methylpropyl)butanoic acid).

### 3. Treatment for Reduction of Loss of Pain Sensitivity

Also described is a use for reducing the loss of pain sensitivity in a subject afflicted with a neuropathy. In a preferred embodiment, the neuropathy is diabetic neuropathy. In a preferred embodiment, the loss of pain sensitivity is a loss in thermal pain sensitivity. This invention contemplates both prophylactic and therapeutic treatment.

In prophylactic treatment, a neublastin polypeptide is administered to a subject at risk of developing loss of pain sensitivity; such subjects would be expected to be subjects with an early stage neuropathy. The treatment with neublastin under such circumstances would serve to treat at-risk patients preventively.

In therapeutic treatment, it is also described that a neublastin polypeptide is administered to a subject who has experienced loss of pain sensitivity as a result of affliction with a neuropathy; such subjects would be expected to be subjects with a late stage neuropathy. The treatment with neublastin under such circumstances would serve to rescue appropriate pain sensitivity in the subject.

### 4. Treatment of Viral Infections and Viral-Associated Neuropathies

Prophylactic treatment of infectious and viral neuropathies is contemplated. Prophylactic treatment is indicated after determination of viral infection and before onset of neuropathic pain. During treatment, NBN polypeptide is administered to prevent appearance of neuropathic pain associated with leprosy, Lyme disease, neuropathic pain associated with infection by a virus, particularly a virus selected from the group consisting of a herpes virus (and more particularly by a herpes zoster virus, which may lead to post-herpetic neuralgia), a human immunodeficiency virus (HIV), and a papilloma virus). In an alternative embodiment, NBN polypeptide is used to reduce the severity of neuropathic pain, should it appear.

Symptoms of acute viral infection often include the appearance of a rash. Other symptoms include, for example, the development of persistent pain in the affected area of the body, which is a common complication of a herpes zoster infection (shingles). Post-herpetic neuralgia can last for a month or more, and may appear several months after any rash-like symptoms have disappeared. Post-herpetic neuralgia may be very severe and prolonged, and can be very resistant to treatment.

### 5. Treatment of Diabetic Neuropathies

Prophylactic treatment of diabetes associated neuropathies is contemplated. Prophylactic treatment of diabetic neuropathies would commence after determination of the initial diagnosis of diabetes or diabetes-associated symptoms and before onset of neuropathic pain. Prophylactic treatment of diabetic neuropathies may also commence upon determining that a subject is at risk for developing diabetes or diabetes-associated symptoms. During treatment, NBN polypeptide is used to prevent appearance of neuropathic pain or reduce the severity of neuropathic pain, should it appear.

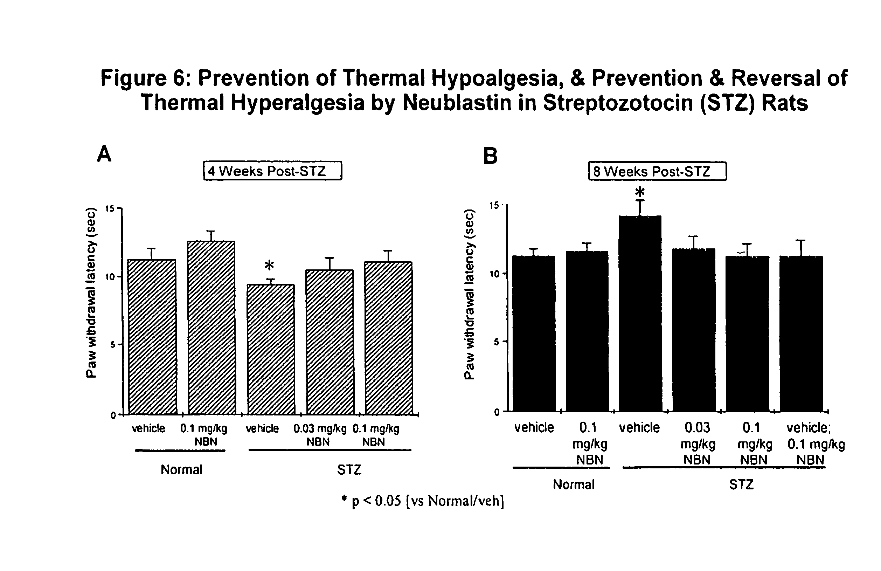

Results in FIG. 6A and FIG. 6B are relevant to treatment of diabetic neuropathy in human patients in need of such treatment. Prevention of thermal hypoalgesia and prevention and reversal of thermal hyperalgesia are contemplated, as described in Example 6.

### 6. Dosage

The foregoing uses contemplate, preferably as systemic administration , a formulation comprising a neublastin polypeptide at a dosage of between 1 µg/kg to 30,000 µg/kg body weight of the subject; per dose; preferably the dosage is between 10 µg/kg to 10,000 µg/kg body weight of the subject, per dose; most preferably the dosage is between 25 µg/kg to 3,000 µg/kg body weight of the subject, per dose.

Various dosing regimes for treatment or prevention of tactile allodynia are contemplated. In one embodiment, a formulation comprising a neublastin polypeptide is applied in the uses of administering to a subject at a dosage of between 1 µg/kg to 30,000 µg/kg body weight of the subject, per dose. In another embodiment, the dosage is between 10 µg/kg to 30,000 µg/kg body weight of the subject, per dose. In a further embodiment, the dosage is between 10 µg/kg to 10,000 µg/kg body weight of the subject, per dose. In a different embodiment, the dosage is between 25 µg/kg to 10,000 µg/kg body weight of the subject, per dose. In yet another embodiment, the dosage is between 25 µg/kg to 3,000 µg/kg body weight of the subject, per dose. In a most preferable embodiment, the dosage is between 50 µg/kg to 3,000 µg/kg body weight of the subject, per dose.

Likewise, various dosing schemes for a treatment for modulating loss of pain sensitivity are described. Administering to a subject a formulation comprising a neublastin polypeptide includes administering NBN at a dosage of between 1 µg/kg to 30,000 µg/kg body weight of the subject, per dose; preferably the dosage is between 10 µg/kg to 10,000 µg/kg body weight of the subject, per dose; most preferably the dosage is between 25 µg/kg to 3,000 µg/kg body weight of the subject, per dose.

### 7. Delivery

The neublastin polypeptide applied in the foregoing uses can be administered via any suitable delivery system, and preferably from the group consisting of intravenous delivery, intramuscular delivery, intrapulmonary delivery, subcutaneous delivery, and intraperitoneal delivery, most preferably via intramuscular delivery, intravenous delivery, or subcutaneous delivery. The neublastin polypeptide applied in the foregoing uses can also be administered via intrathecal delivery.

### 8. Formulation

This invention also discloses novel pharmaceutical compositions comprising a therapeutically effective amount of neublastin polypeptides suspended, dissolved, or dispersed in a pharmaceutically accepted carrier for treating, preventing or delaying neuropathic pain, wherein the neuropathic pain is allodynia, hyperalgesic pain or phantom pain.

For use in therapy the polypeptide of the invention may be administered in any convenient form. In a preferred embodiment, the polypeptide of the invention is incorporated into a pharmaceutical composition together with one or more adjuvants, excipients, carriers and/or diluents, and the pharmaceutical composition prepared by the skilled person using conventional methods known in the art. Further details on techniques for formulation and administration may be found in the latest edition of REMINGTON'S PHARMACEUTICAL SCIENCES (Maack Publishing Co., Easton, PA). Acceptable diluents, carriers and excipients typically do not adversely affect the recipient's homeostasis, particularly electrolyte balance. Acceptable carriers can include biocompatible, inert or bioabsorbable salts, buffering agents, oligo- or polysaccharides, polymers, viscosity-improving agents, preservatives and the like. One exemplary carrier comprises normal physiologic saline (0.15 M NaCl, pH 7.0 to 7.4). Another exemplary carrier comprises 50 mM sodium phosphate, 100 mM sodium chloride.

### 9. Regimes

The frequency of dosing for the neublastin polypeptides of this invention is within the skills and clinical judgement of physicians. Typically, the administration regime is established by clinical trials which may establish optimal administration parameters. However, the practitioner may vary such administration regimes according to the subject's age, health, weight, sex and medical status. The frequency of dosing may also vary between acute and chronic treatments for neuropathy. In addition, the frequency of dosing may be varied depending on whether the treatment is prophylactic or therapeutic.

### EXAMPLES

### EXAMPLE 1: Expression of Neublastin Polypeptide

### A. Expression in E. coli

For expression and purification in bacteria, a plasmid encoding rat neublastin was expressed in *E*. *coli* as a His-tagged fusion protein with an enterokinase cleavage site immediately adjacent to the start of the mature 113 amino acid NBN sequence. The *E. coli* cells were grown in a 500 L fermentor and frozen cell paste was provided. The *E. coli* cells were lysed in a Manton Gaulin Press and the rat NBN was recovered from the insoluble washed pellet fraction.

The NBN was extracted from the pellet with guanidine hydrochloride, refolded, and the His-tag removed with enterokinase. For further purification, the product was then subjected to chromatography on Ni NTA agarose (Qiagen), and to chromatography on Bakerbond WP CBX cation exchange resin.

The resulting product was subjected to extensive characterization including analysis by SDS-PAGE, size exclusion chromatography (SEC), reverse phase HPLC, matrix assisted laser desorption/ionization mass spectrometry (MALD/IMS), peptide mapping, assessment of activity in the KIRA ELISA, and determination of endotoxin content. The purity of the NBN product as measured by SDS-PAGE and SEC was greater than 95%. The NBN product migrated under non-reducing conditions as a dimer, consistent with its predicted structure. The endotoxin content of the material is routinely less than 1 EU/mg. The specific activity of the NBN in the KIRA ELISA is approximately 10 nM. The product was formulated at 1 mg/mL in PBS pH 6.5. The material can be supplied as a frozen liquid, which is stored at -70°C.

Similar expression systems have also been constructed for human NBN. From these constructs, human NBN has been expressed in *E. coli*.

### B. Expression in Mammalian Cells

**Construction of plasmid pJC070.14** In order to express the neublastin cDNA in Chinese hamster ovary cells, a cDNA fragment encoding the prepro form of human neublastin was inserted into the mammalian expression vector pEAG347 to generate plasmid pJC070.14. pEAG347 contains tandem SV40 early and adenovirus major late promoters (derived from plasmid pAD2beta; Norton and Coffin, Mol. Cell. Biol. 5: 281 (1985)), a unique NotI cloning site, followed by SV40 late transcription termination and polyA signals (derived from plasmid pCMVbeta; MacGregor and Caskey, Nucl. Acids. Res. 17: 2365 (1989)). In addition, pEAG347 contains a pUC19-derived plasmid backbone and a pSV2dhfr-derived dhfr for MTX selection and amplification in transfected CHO cells.

Plasmid pJC070.14 was generated in two steps. First, a fragment encoding the prepro form of human neublastin was isolated from plasmid pUbilZ-NBN using the polymerase chain reaction with oligonucleotides KD2-824 5'AAGGAAAAAA GCGGCCGCCA TGGAACTTGG ACTTGGAGG3' (SEQ ID NO:9), KD2-825 5'TTTTTTCCTT GGCGGCCGCT CAGCCCAGGC AGCCGCAGG3' (SEQ ID NO:10) and PFU polymerase. The fragment was cloned into the Srf-1 site of pPCR-Script Amp SK(+) to generate the plasmid pJC069. In the second step, a partial Not-1 digest was performed on plasmid pJC069 to generate a 685bp fragment (containing the neublastin gene) which was cloned into the Not-1 site of plasmid pEAG347 to generate plasmid pJC070.14. Transcription of the neublastin gene in plasmid pJC070.14 is controlled by the adenovirus major late promoter.

**Generation of CHO cell lines expressing Neublastin.** 200 µg of pJC070.14 was linearized by digestion with the restriction endonuclease Mlu-1. The DNA was extracted with phenol: chloroform:isoamyl alcohol (25:24:1) and ethanol precipitated. The linearized DNA was resuspended in 20mM Hepes pH7.05, 137mM NaCl, 5mM KCl, 0.7mM Na₂HPO₄, 6mM dextrose (HEBS) and introduced into ~4E7 CHO dukx B1(*dhfr-*) cells (p23) by electroporation (280V and 960 µF). Following electroporation, the cells were returned to culture in α+ Modified Eagle's Medium (MEM) supplemented with 10% fetal bovine serum (FBS) for two days. The cells were then trypsinized and replated in 100 mm dishes (100,000 cells/plate) in α-MEM (lacking ribo- and deoxyribonucleosides), supplemented with 10% dialyzed FBS, for five days. The cells were subsequently split at a density of 100,000 cells/100mm plate, and selected in 200nM methotrexate. Resistant colonies were picked and scaled up to 6 well plates; conditioned media from each clone was screened using a specific assay for neublastin described below. The twelve clones expressing the highest level of neublastin were scaled up to T162 flasks and subsequently reassayed. These CHO cell lines produced neublastin in the range of 25 to 50 ng/ml/day.

**Ternary complex assay for neublastin.** The presence of neublastin was assessed in the media of CHO cell line supernatants using a modified form of a ternary complex assay described by Sanicola et al. (Proc Natl Acad Sci USA 94: 6238 (1997).

**Similar mammalian expression constructs have been made and similar studies have been performed for both human NBN and rat NBN.** *In vivo* testing of NBN activity in rats have been performed. Rat studies almost exclusively were performed with rat NBN.

### EXAMPLE 2: Efficacy of Full Length Neublastin in a Nerve Ligation Animal Model of Neuropathic Pain - Prevention of Neuropathic Pain

The preventive effect of neublastin on tactile allodynia and thermal hyperalgesia was studied in the Chung L5/L6 spinal nerve ligation ("SNL") model (Kim and Chung (1992), Pain 50: 355-363. Sprague-Dawley male rats (250-300 g) were divided into four groups. One group of rats (n=7) received a sham operation, and were administered vehicle by subcutaneous injection. A second group of rats (n=7) received a sham operation and were administered rat Neublastin (1mg/kg) by subcutaneous injection. A third group of rats (n=7) received the spinal nerve ligation, and were administered vehicle by subcutaneous injection. A fourth group of rats (n=7) received the spinal nerve ligation, and were administered rat neublastin (1 mg/kg) by subcutaneous injection. The vehicle consisted of 5mM phosphate and 150mM sodium chloride at pH 6.5. Neublastin or vehicle was injected 30 minutes before the spinal nerve ligation or sham operation, and then on days 2, 4, 7, 9, 11 and 14 following the ligation or operation (post-SNL). The Von Frey (Chaplan et al. (1994), J. Neurosci. Meth. 53: 55-63) and Hargreave's (Hargreaves et al. (1988), Pain 32: 77-88) behavioral tests were used to monitor tactile and thermal responses, respectively. These pain responses were monitored prior to the spinal nerve ligation or sham operation to establish baseline responses, and then daily for two weeks following the operation.

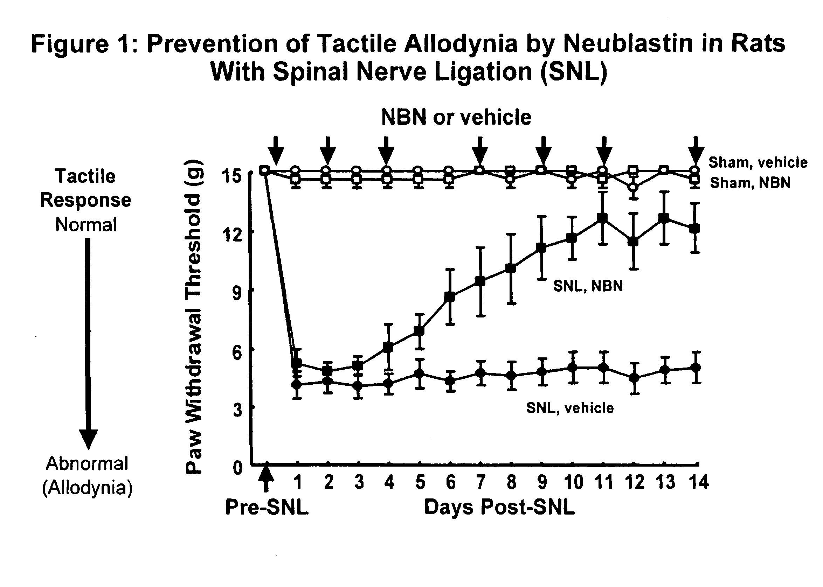

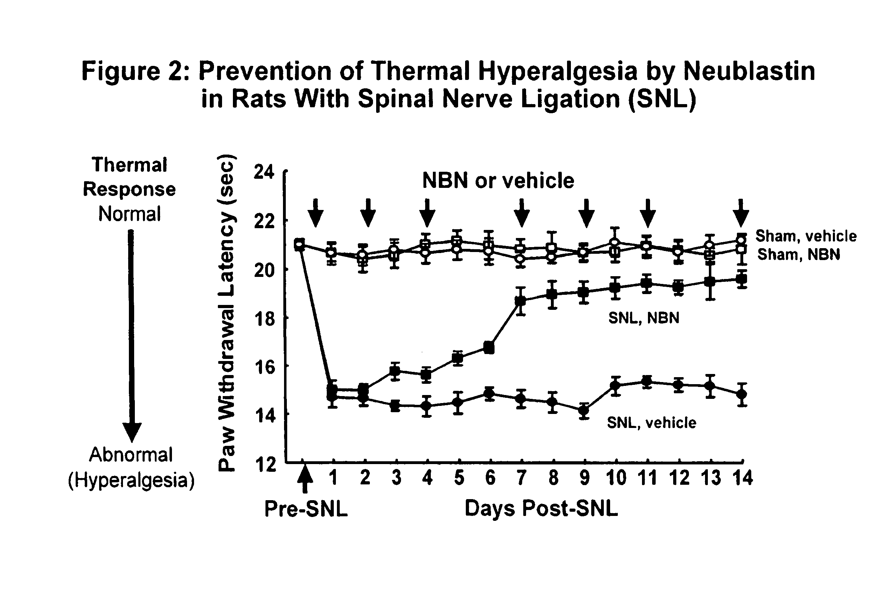

The results are depicted in FIGS. 1 and 2 as averages ± standard errors of the mean. Subcutaneous administration of neublastin (as denoted by downward arrows in FIGS. 1 and 2) led to nearly complete normalization of both types of neuropathic pain (tactile, FIG. 1 and thermal, FIG. 2) in rats with spinal nerve ligation. In sham operated rats, subcutaneous administration of neublastin did not significantly alter tactile (FIG. 1) or thermal sensitivity (FIG. 2). In spinal nerve ligated rats, the effect of neublastin on thermal sensitivity first became evident 3 days after the initiation of neublastin administration, whereas the effect on tactile allodynia first became evident slightly later, at 4-5 days after the initiation of neublastin administration. The effect of neublastin on thermal sensitivity reached a plateau approximately 7-8 days after the initiation of neublastin administration whereas the effect on tactile allodynia reached a plateau on approximately 10-11 days after the initiation of neublastin administration. The effects of neublastin did not diminish during the 2-3 day interval between administrations. In fact, there was substantial normalization of both pain behaviors between the administrations of neublastin on days 4 and 7, as measured on days 5 and 6. Following the administration of neublastin on day 11, the extant maximal normalization of both pain behaviors remained constant, suggesting that the normalization effect of neublastin on neuropathic pain is maintained for at least 3 days.

### EXAMPLE 3: Treatment of Neuropathy With a Truncated Neublastin Polypeptide

The preventive effect of a polypeptide containing the carboxy terminal 102 amino acids of mature rat neublastin in treating tactile allodynia and thermal hyperalgesia, two peripheral neuropathic conditions, is demonstrated in a Chung L5/L6 spinal nerve ligation ("SNL") model.

Sprague-Daley male rats (approximately 250-300 grams) are divided into four groups. One group of rats (n=6) receive a sham operation and are administered vehicle by subcutaneous injection. A second group of rats (n=6) receive a sham operation and are administered truncated neublastin (1 mg/kg) by subcutaneous injection. A third group of rats (n =6) receive a spinal nerve ligation and are administered vehicle by subcutaneous ligation. A fourth group (n=6) receives the spinal nerve ligation, and are administered truncated neublastin (1 mg/kg) by subcutaneous injection. The vehicle includes 5 mM phosphate and 150 mM sodium chloride at pH 6.5. Truncated neublastin or vehicle is administered on days 0, 2, 4, 7, 9, 11 and 14. On day 0, truncated neublastin is administered 30 minutes before the spinal nerve ligation or sham operation. Pain responses are determined using behavioral tests as described in Chaplan et al. (1994), J. Neurosci. Meth. 53:55-63 and Hargreaves et al. (1988), Pain 32:77-88. These tests monitor tactile and thermal responses, respectively. The pain responses are monitored prior to the spinal nerve ligation or sham operation to establish baseline responses. Pain responses are then monitored daily for two weeks following the operation.

Subcutaneous administration of truncated neublastin is expected to lead to normalization of both types of neuropathic pain in rats with spinal nerve ligation. In sham operated rats, subcutaneous administration of truncated neublastin is not expected to significantly alter tactile or thermal sensitivity. The effect of neublastin on thermal sensitivity is predicted to become evident about 3 days after the administration of neublastin, whereas the effect on tactile allodynia is expected to first become evident slightly later.

### EXAMPLE 4: Neublastin Efficacy in a Nerve Ligation Animal Model of Neuropathic Pain - Reversal of Neuropathic Pain

The reversal effect of neublastin on tactile allodynia and thermal hyperalgesia was studied in the Chung L5/L6 spinal nerve ligation ("SNL")- model. Sprague-Dawley male rats (270 - 275g) were divided into four groups. One group of rats (n=8) received a sham operation, and were administered vehicle by subcutaneous injection. A second group of rats (n=8) received a sham operation and were administered rat neublastin (1mg/kg) by subcutaneous injection. A third group of rats (n=8) received the spinal nerve ligation, and were administered vehicle by subcutaneous injection. A fourth group of rats (n=8) received the spinal nerve ligation, and were administered rat neublastin (1 mg/kg) by subcutaneous injection. The vehicle consisted of 5mM phosphate and 150mM sodium chloride at pH 6.5. The Von Frey (Chaplan et al. (1994), J. Neurosci. Meth. 53: 55-63) and Hargreave's (Hargreaves et al. (1988), Pain 32: 77-88) behavioral tests were used to monitor tactile and thermal responses, respectively. These pain responses were monitored prior to the spinal nerve ligation or sham operation to establish baseline responses, and then daily for 2 weeks following the operation. Neublastin or vehicle was injected 60 minutes before behavioral testing on days 3, 5, 7, 10, 12 and 14 following the ligation or operation (post-SNL).

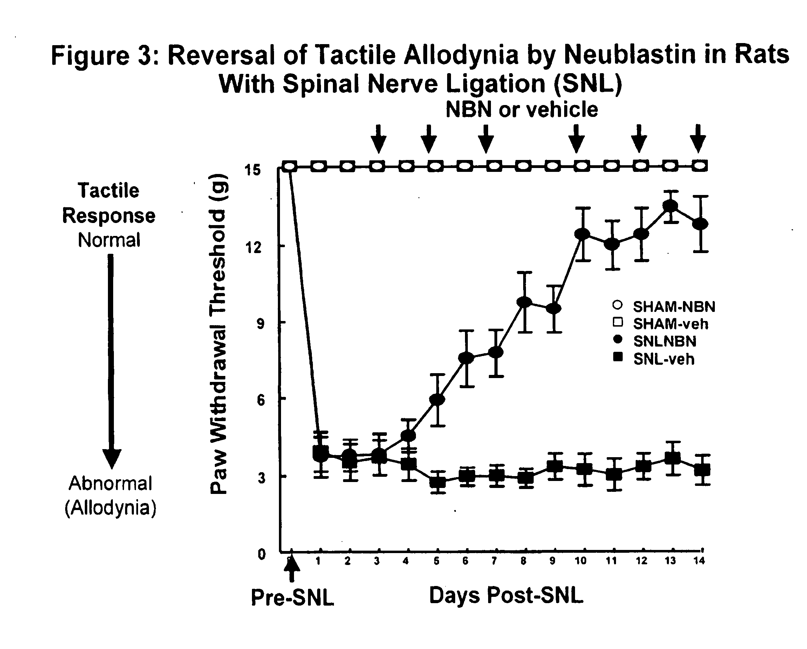

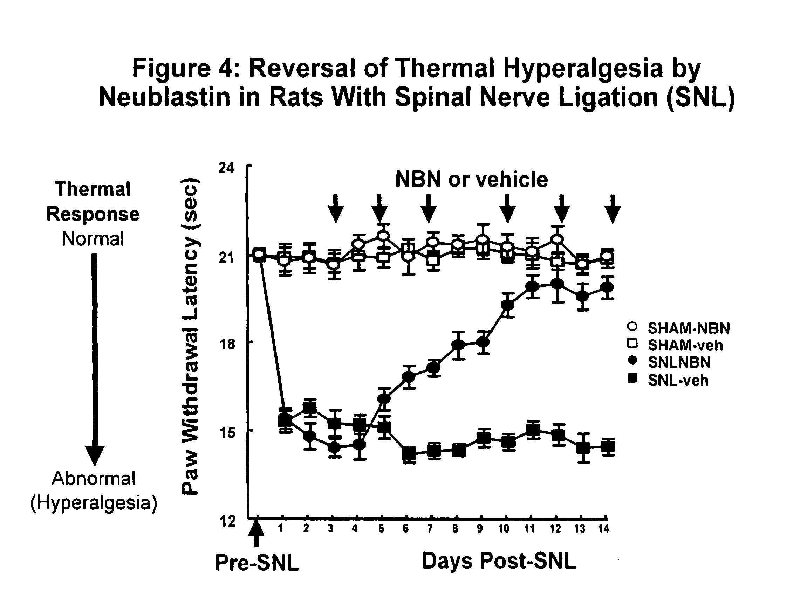

The results are depicted in FIGS. 3 and 4 as averages ± standard errors of the mean. Both types of neuropathic pain behavior (tactile allodynia shown in FIG. 3, and thermal hyperalgesia shown in FIG. 4) developed fully by day 3, as expected. Subcutaneous administration of neublastin (as denoted by downward arrows in FIGS. 3 and 4) led to nearly complete reversal of both types of neuropathic pain (tactile in FIG. 3 and thermal in FIG. 4) in rats with spinal nerve ligation, so that tactile and thermal responses were normalized. In sham operated rats, subcutaneous administration of neublastin did not significantly alter tactile (FIG. 3) or thermal (FIG. 4) sensitivity. In rats with spinal nerve ligation, the effect of neublastin on thermal sensitivity and tactile allodynia first became evident 2 to 3 days after the initiation of neublastin administration. The effect of neublastin on thermal sensitivity and tactile allodynia reached a plateau approximately 7-8 days after the initiation of neublastin administration. The effects of neublastin did not diminish during the 2 to 3 day interval between administrations. In fact, there was substantial normalization of both pain behaviors between the administrations of neublastin on days 3, 5, 7 and 10.

### EXAMPLE 5: Neublastin Efficacy in a Streptozotocin Animal Model of Neuropathic Pain - Prevention of Loss of Pain Sensitivity (Illustrative Example)

The preventive effect of neublastin on loss of thermal sensitivity (thermal hypoalgesia) was studied in the streptozotocin ("STZ") model of diabetic neuropathy. Sprague-Dawley female rats (250 - 275g) were divided into 4 groups, each comprised of 10 animals. One group of rats did not receive STZ; these rats received vehicle by subcutaneous injection. Three groups of rats received 1 injection of STZ (50 mg/kg in sterile saline) and were subsequently confirmed to be hyperglycemic by spectophotometric assay of blood samples. Of the 3 groups of hyperglycemic rats, 1 group received vehicle by subcutaneous injection, a second group was administered rat neublastin (0.1 mg/kg) by subcutaneous injection, and a third group of rats was administered rat neublastin (1mg/kg) by subcutaneous injection. The vehicle consisted of 5mM phosphate and 150mM sodium chloride at pH 6.5. Neublastin or vehicle was administered 3 times per week (Monday, Wednesday, Friday schedule) for 8 weeks, and was initiated at the time of STZ induction of hyperglycemia. Thermal response latencies were assessed after 8 weeks as described in Calcutt et al. (2000), Anesthesiology 93: 1271-1278. In brief, a radiant heat source was applied to the plantar surface of the paw so that the surface temperature increased from 30°C to 38.5°C in 20 seconds, and the time latency between initiation of heating and paw withdrawal was measured to assess thermal response latency. An increase in paw withdrawal latency indicates a loss of thermal sensitivity (thermal hypoalgesia).

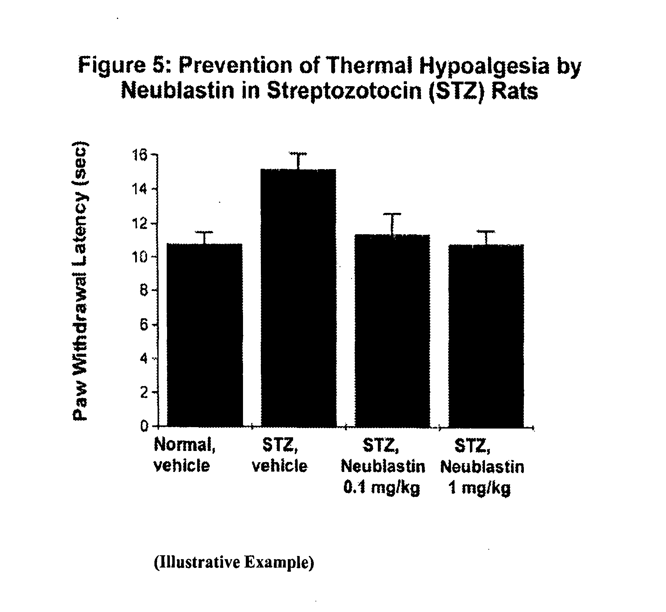

The results are depicted in FIG. 5 as averages ± standard errors of the mean. As expected at 8 weeks post-STZ, the paw withdrawal latency increased in vehicle treated STZ rats (STZ vehicle) compared to normal rats, indicating that thermal hypoalgesia had been induced by the STZ injection. Administration of neublastin prevented the increase in thermal response latency in hyperglycemic (STZ) rats. Both doses of neublastin (1 mg/kg and 0.1 mg/kg) nearly completely normalized thermal sensitivity after 8 weeks of administration at 3 times per week. These results demonstrate that neublastin prevents thermal hypoalgesia, the loss of thermal sensitivity that occurs in the STZ rat model of diabetic neuropathy.

### EXAMPLE 6: Prevention of Thermal Hypoalgesia, and Prevention and Reversal of Thermal Hyperalgesia by Neublastin in Streptozotocin Rats.

Rat NBN 113 was prepared in *E*. *coli* as described in Example 1. The preventive effect of neublastin on loss of thermal sensitivity (thermal hypoalgesia), and prevention and reversal by neublastin of increased thermal sensitivity (thermal hyperalgesia) was studied in the streptozotocin ("STZ") rat model of diabetic neuropathy, as described in Example 5. Dosage studies described in Example 5 were confirmed and extended upon. Results are shown in FIG. 6A and FIG. 6B.

The results in FIG. 6A and FIG. 6B are depicted as averages ± standard errors of the mean. As expected at 4 weeks post-STZ, the paw withdrawal latency decreased in vehicle-treated STZ rats, as compared to normal rats, indicating that thermal hyperalgesia had been induced by the STZ injection. All doses of subcutaneous neublastin (0.03 mg/kg and 0.1 mg/kg) prevented thermal hyperalgesia in STZ rats after 4 weeks of administration at 3 times per week, as shown in FIG. 6A. All doses of subcutaneous neublastin (0.03 mg/kg and 0.1 mg/kg) prevented thermal hypoalgesia in STZ rats after 8 weeks of administration at 3 times per week, as shown in FIG. 6B. In addition, as shown in FIG. 6B, subcutaneous neublastin (0.1 mg/kg) administered during the second 4 wks of the 8 week treatment study (veh; 0.1 mg/kg NBN) not only reversed the thermal hyperalgesia that was apparent at 4 weeks post STZ treatment, but also prevented thermal hypoalgesia at 8 weeks from developing in STZ rats. These results demonstrate that neublastin prevents and reverses thermal hyperalgesia in the STZ rat model of diabetic neuropathy, and that neublastin prevents the loss of thermal sensitivity (thermal hypoalgesia) that occurs in the STZ rat model of diabetic neuropathy.

### EXAMPLE 7: Neublastin Efficacy in a Nerve Ligation Animal Model of Neuropathic Pain - Reversal of Neuropathic Pain is Dose Dependent.

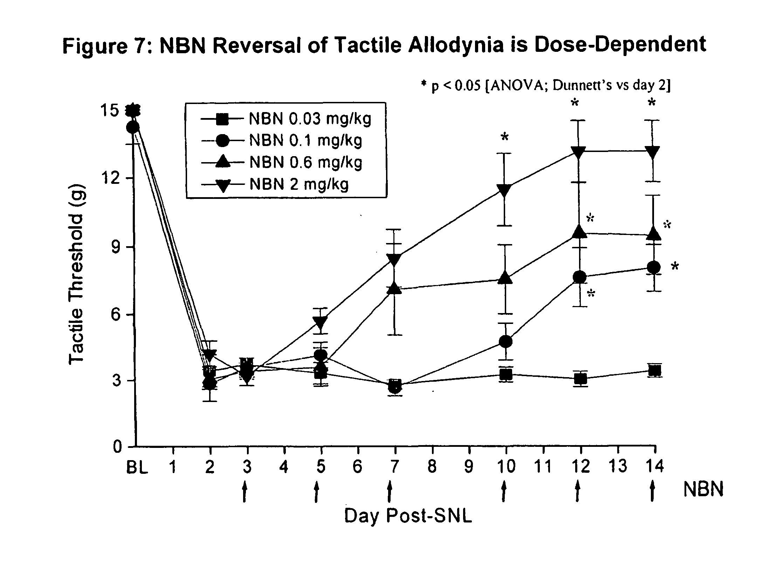

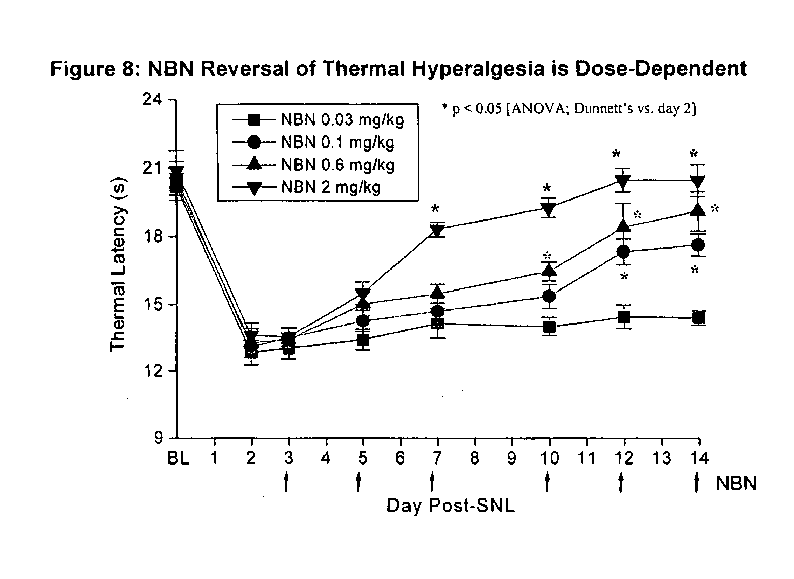

Rat NBN 113 was prepared in *E. coli* as described in Example 1. Analysis of dose-dependent reversal by NBN 113 of tactile allodynia and thermal hyperalgesia was performed in rats using the Chung L5/L6 spinal nerve ligation ("SNL") animal model, as described in Example 4, with NBN doses of 0.03 mg/kg, 0.1 mg/kg, 0.6 mg/kg and 2 mg/kg. Experimental results shown in FIGS 3 and 4 were confirmed and expanded upon. Results are shown in FIG. 7 and FIG. 8.

The results in FIGS. 7 and 8 are depicted as averages ± standard errors of the mean. BL indicates baseline responses. Both types of neuropathic pain behavior (tactile allodynia shown in FIG. 7, and thermal hyperalgesia shown in FIG. 8) developed fully by day 2, as expected. Subcutaneous administration of 2 mg/kg neublastin (NBN) 3 times per week (as denoted by the arrows) led to nearly complete reversal of both types of neuropathic pain (tactile in FIG. 7 and thermal in FIG. 8) in rats with spinal nerve ligation, so that tactile and thermal responses were normalized. As shown in FIG. 7, neublastin reversal of spinal nerve ligation-induced tactile allodynia was dose-dependent. Neublastin (NBN) dosing at 0.1 mg/kg s.c. or 0.6 mg/kg s.c. partially reversed tactile allodynia after 9 (as well as 11) days of dosing three times per week, whereas NBN at 2 mg/kg s.c. significantly reversed tactile allodynia after 7 (as well as 9 and 11) days of dosing three times per week, with nearly complete reversal of tactile allodynia after 9 and 11 days of dosing three times per week. Moreover, the mean reversal of spinal nerve ligation-induced tactile allodynia on day 14 post-SNL was greater with increasing subcutaneous doses of neublastin from 0.1 mg/kg to 0.6 mg/kg to 2 mg/kg. Neublastin (NBN) at 0.03 mg/kg s.c. did not significantly reverse spinal nerve ligation-induced tactile allodynia during 11 days of dosing at three times per week.

As depicted in FIG. 8, Neublastin (NBN) reversal of spinal nerve ligation (SNL) - induced thermal hyperalgesia was also dose-dependent. NBN dosing at 0.1 mg/kg significantly reversed thermal hyperalgesia after 9 (as well as 11) days of dosing three times per week, NBN at 0.6 mg/kg significantly reversed thermal hyperalgesia after 7 (as well as 9 and 11) days of dosing three times per week, and NBN at 2 mg/kg significantly reversed thermal hyperalgesia after 4 (as well as 7, 9 and 11) days of dosing three times per week. Moreover, the mean reversal of spinal nerve ligation-induced thermal hyperalgesia on day 14 post-SNL was greater with increasing subcutaneous doses of neublastin from 0.1 mg/kg to 0.6 mg/kg to 2 mg/kg. NBN at 0.03 mg/kg s.c. did not significantly reverse spinal nerve ligation-induced thermal hyperalgesia during 11 days of dosing at three times per week.

## Claims

1. Use of a neublastin polypeptide for the preparation of a pharmaceutical composition for treating, preventing, or delaying neuropathic pain, wherein the neuropathic pain is allodynia, hyperalgesic pain or phantom pain.

2. A neublastin polypeptide for use in the treatment, prevention, or delay of neuropathic pain, wherein the neuropathic pain is allodynia, hyperalgesic pain or phantom pain.

3. The use of claim 1 or the neublastin polypeptide for use as in claim 2, wherein the pharmaceutical composition or the neublastin polypeptide is prepared for administration via systemic delivery

4. The use of claim 1 or the neublastin polypeptide for use as in claim 2, wherein the pharmaceutical composition or the neublastin polypeptide is prepared for administration via intravenous delivery.

5. The use of claim 1 or the neublastin polypeptide for use as in claim 2, wherein the pharmaceutical composition or the neublastin polypeptide is prepared for administration via subcutaneous delivery.

6. The use of claim 1 or the neublastin polypeptide for use as in claim 2, wherein the neuropathic pain is allodynia.

7. The use of claim 1 or the neublastin polypeptide for use as in claim 2, wherein the allodynia is tactile allodynia.

8. The use of claim 1 or the neublastin polypeptide for use as in claim 2, wherein the neuropathic pain is hyperalgesic pain.

9. The use or the neublastin polypeptide for use as in claim 8, wherein the hyperalgesic pain is thermal hyperalgesic pain.

10. The use of claim 1 or the neublastin polypeptide for use as in claim 2, wherein the neuropathic pain is associated with post-herpetic neuralgia, diabetic neuropathy, or sciatica.

11. The use of claim 1 or the neublastin polypeptide for use as in claim 2, wherein said neuropathic pain is associated with infection by a virus.

12. The use or the neublastin polypeptide for use as in claim 11, wherein said virus is selected from the group consisting of a herpes virus, a human immunodeficiency virus (HIV), and a papilloma virus.

13. The use of claim 1 or the neublastin polypeptide for use as in claim 2, wherein said neuropathic pain is neuropathic pain associated with administration of a therapeutic agent.

14. The use or the neublastin polypeptide for use as in claim 13, wherein said therapeutic agent is an anti-cancer agent.

15. The use or the neublastin polypeptide for use as in claim 14, wherein said anti-cancer agent is selected from the group consisting of taxol, taxotere, cisplatin, nocodazole, vincristine, vindesine, and vinblastine.

16. The use or the neublastin polypeptide for use as in claim 13, wherein said therapeutic agent is an anti-viral agent.

17. The use or the neublastin polypeptide for use as in claim 16, wherein said anti-viral agent is selected from the group consisting of ddI, DDC, d4T, foscarnet, dapsone, metronidazole, and isoniazid.

18. The use of claim 1 or the neublastin polypeptide for use as in claim 2, wherein the neuropathic pain is associated with hereditary neuropathy (including but not limited to Friedreich ataxia, familial amyloid polyneuropathy, Tangier disease, Fabry disease), metabolic disorders (including but not limited to renal insufficiency and hypothyroidism), vitamin deficiencies (including but not limited to vitamin B12 deficiency, vitamin B6 deficiency, and vitamin E deficiency), toxic and iatrogenic neuropathies (including but not limited to alcoholism, vitamin B6 intoxication, hexacarbon intoxication, amiodarone, chloramphenicol, disulfiram, isoniazide, gold, lithium, metronidazole, misonidazole, nitrofurantoin), infectious neuropathies (including but not limited to leprosy and Lyme disease), auto-immune neuropathies (including but not limited to Guillain-Barre syndrome, chronic inflammatory de-myelinating polyneuropathy, monoclonal gammopathy of undetermined significance, and polyneuropathy), trigeminal neuralgia, entrapment syndromes (including but not limited to Carpel tunnel), post-traumatic neuralgia, phantom limb pain, multiple sclerosis pain, complex regional pain syndromes (including but not limited to reflex sympathetic dystrophy and causalgia), neoplasia, vasculitic/angiopathic neuropathy, and idiopathic neuropathy.

19. The use or the neublastin polypeptide for use according to any one of the preceding claims, wherein the neublastin polypeptide is modified with a derivative moiety to have .an extended residence time or increased concentration in body fluids.

20. The use or the neublastin polypeptide for use as in claim 19, wherein said derivative moiety is selected from the group consisting of a polyethylene glycol moiety, aliphatic esters, amides, N-acyl-derivatives, and O-acyl derivatives.

21. The use or the neublastin polypeptide for use according to any one of the preceding claims, wherein the amino acid sequence of said neublastin polypeptide comprises a polypeptide selected from the group consisting of:
a. at least one polypeptide comprising AA₋₈₀-AA₁₄₀ of SEQ ID NO:2, AA₋₄₁-AA₁₄₀ of SEQ ID NO:2, AA₁-AA₁₄₀ of SEQ ID NO:2, AA₂₅-AA₁₄₀ of SEQ ID NO:2, AA₂₈-AA₁₄₀ of SEQ ID NO:2, AA₋₈₀-AA₁₄₄ of SEQ ID NO:4, AA₁-AA₁₄₄ of SEQ ID NO:4, AA₁-AA₂₂₄ of SEQ ID NO:5, or AA₈₁-AA₂₂₄ of SEQ ID NO:5;
b. at least one polypeptide comprising the C-terminal sequence set forth in either AA₁₀₇-AA₁₄₀ of SEQ ID NO:2 or AA₇₆-AA₁₄₀ of SEQ ID NO:2, and which retain the seven Cys residues characteristic of the GDNF family and of the TGF-β super family;
c. at least one polypeptide comprising SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 or SEQ ID NO:27; and
d. at least one polypeptide sequence that has greater than 70%, more preferably 85%, still more preferably 90%, and still further preferably 95% amino acid homology to any one of the sequences in (a) - (c) above.

22. The use or the neublastin polypeptide for use according to any one of the preceding claims, wherein the amino acid sequence of said neublastin polypeptide comprises a polypeptide selected from the group consisting of at least one polypeptide comprising AA₄₂-AA₁₄₀ of SEQ ID NO:2 or AA₃₇-AA₁₄₀ of SEQ ID NO:2.

23. The use or the neublastin polypeptide for use according to any one of the preceding claims, wherein the pharmaceutical composition further comprises an analgesia-inducing compound selected from the group consisting of opioids, anti-arrhythmics, topical analgesics, local anaesthetics, anticonvulsants, antidepressants, corticosteroids, and NSAIDS.

24. The use or the neublastin polypeptide for use as in claim 23, wherein the analgesia-inducing compound is an anticonvulsant.

25. The use or the neublastin polypeptide for use as in claim 23, wherein the analgesia-inducing compound is gabapentin (1-(aminomethyl)cyclohexane acetic acid) or pregabalin (S-(+)-4-amino-3-(2-methylpropyl)butanoic acid).

26. The use or the neublastin polypeptide for use according to any one of the preceding claims, wherein the dosage of the neublastin polypeptide is between 1 µg/kg to 30,000 µg/kg body weight of the subject, per dose, between 10 µg/kg to 10,000 µg/kg body weight of the subject, per dose, or between 25 µg/kg to 3,000 µg/kg body weight of the subject, per dose.

27. The use or the neublastin polypeptide for use according to any one of the preceding claims, wherein the pharmaceutical composition is a timed-release composition.

## Patentansprüche

1. Verwendung eines Neublastin-Polypeptids zur Herstellung einer pharmazeutischen Zusammensetzung zur Behandlung, Vorbeugung oder Verzögerung neuropathischer Schmerzen, wobei es sich bei den neuropathischen Schmerzen um Allodynie, hyperalgetische Schmerzen oder Phantomschmerzen handelt.

2. Ein Neublastin-Polypeptid zur Verwendung bei der Behandlung, Vermeidung oder Verzögerung neuropathischer Schmerzen, wobei es sich bei den neuropathischen Schmerzen um Allodynie, hyperalgetische Schmerzen oder Phantomschmerzen handelt.

3. Verwendung nach Anspruch 1 oder das Neublastin-Polypetid zur Verwendung wie in Anspruch 2, wobei die pharmazeutische Zusammensetzung oder das Neublastin-Polypeptid zur Verabreichung mittels systemischer Gabe hergerichtet ist.

4. Verwendung nach Anspruch 1 oder das Neublastin-Polypeptid zur Verwendung wie in Anspruch 2, wobei die pharmazeutische Zusammensetzung oder das Neublastin-Polypeptid zur Verabreichung mittels intravenöser Gabe hergerichtet ist.

5. Verwendung nach Anspruch 1 oder das Neublastin-Polypeptid zur Verwendung wie in Anspruch 2, wobei die pharmazeutische Zusammensetzung oder das Neublastin-Polypeptid zur Verabreichung mittels subkutaner Gabe hergerichtet ist.

6. Verwendung nach Anspruch 1 oder das Neublastin-Polypeptid zur Verwendung wie in Anspruch 2, wobei es sich bei den neuropathischen Schmerzen um Allodynie handelt.

7. Verwendung nach Anspruch 1 oder das Neublastin-Polypeptid zur Verwendung wie in Anspruch 2, wobei es sich bei der Allodynie um eine taktile Allodynie handelt.

8. Verwendung nach Anspruch 1 oder das Neublastin-Polypeptid zur Verwendung wie in Anspruch 2, wobei es sich bei den neuropathischen Schmerzen um hyperalgetische Schmerzen handelt.

9. Verwendung oder das Neublastin-Polypeptid zur Verwendung wie in Anspruch 8, wobei es sich bei den hyperalgetischen Schmerzen um thermale hyperalgetische Schmerzen handelt.

10. Verwendung nach Anspruch 1 oder das Neublastin-Polypeptid zur Verwendung wie in Anspruch 2, wobei die neuropathischen Schmerzen mit postherpetischer Neuralgie, diabetischer Neuropathie oder Ischialgie in Zusammenhang stehen.

11. Verwendung nach Anspruch 1 oder das Neublastin-Polypeptid zur Verwendung wie in Anspruch 2, wobei die neuropathischen Schmerzen mit einer Infektion durch ein Virus in Zusammenhang stehen.

12. Verwendung oder das Neublastin-Polypeptid zur Verwendung wie in Anspruch 11, wobei das Virus ausgewählt ist aus der Gruppe bestehend aus einem Herpes-Virus, einem menschlichen Immunschwäche-Virus (HIV) und einem Papillomvirus.

13. Verwendung nach Anspruch 1 oder das Neublastin-Polypeptid zur Verwendung wie in Anspruch 2, wobei die neuropathischen Schmerzen neuropathische Schmerzen sind, die mit Verabreichung eines therapeutischen Mittels in Zusammenhang stehen.

14. Verwendung oder das Neublastin-Polypeptid zur Verwendung wie in Anspruch 13, wobei das therapeutische Mittel ein Antikrebsmittel ist.

15. Verwendung oder das Neublastin-Polypeptid zur Verwendung wie in Anspruch 14, wobei besagtes Antikrebsmittel ausgewählt ist aus der Gruppe bestehend aus Taxol, Taxotere, Cisplatin, Nocodazol, Vincristin, Vindesin und Vinblastin.

16. Verwendung oder das Neublastin-Polypeptid zur Verwendung wie in Anspruch 13, wobei das therapeutische Mittel ein Antivirusmittel ist.

17. Verwendung oder das Neublastin-Polypeptid zur Verwendung wie in Anspruch 16, wobei das Antivirusmittel ausgewählt ist aus der Gruppe bestehend aus ddl, DDC, d4T, Foscarnet, Dapson, Metronidazol und Isoniazid.

18. Verwendung nach Anspruch 1 oder das Neublastin-Polypeptid zur Verwendung wie in Anspruch 2, wobei die neuropathischen Schmerzen in Zusammenhang stehen mit hereditären Neuropathien (einschließlich, aber nicht beschränkt auf Friedreich-Ataxie, familiäre Amyloid-Polyneuropathie, Tangier-Krankheit, Fabry-Krankheit), Stoffwechselkrankheiten (einschließlich, aber nicht beschränkt auf Niereninsuffizienz und Hypothyreose), Vitaminmangel (einschließlich, aber nicht beschränkt auf Vitamin-B12-Mangel, Vitamin-B6-Mangel und Vitamin-E-Mangel), toxischen und iatrogenen Neuropathien (einschließlich, aber nicht beschränkt auf Alkoholismus, Vitamin-B6-Vergiftung, Hexacarbonvergiftung, Amiodaron, Chloramphenicol, Disulfiram, Isoniazid, Gold, Lithium, Metronizadol, Misonidazol, Nitrofurantoin), infektiösen Neuropathien (einschließlich, aber nicht beschränkt auf Lepra und Lyme-Borreliose), Autoimmunneuropathien (einschließlich, aber nicht beschränkt auf das Guillain-Barré-Syndrom, chronische inflammatorische demyelinisierende Polyneuropathie, monoklonale Gammopathie ungeklärter Bedeutung und Polyneuropathie), Trigeminusneuralgie, Entrapment-Syndrom (einschließlich, aber nicht beschränkt auf das Karpaltunnel-Syndrom), posttraumatischen Neuralgien, Phantomschmerzen, Multiple-Sklerose-Schmerzen, komplexem regionalen Schmerzsyndrom (einschließlich, aber nicht beschränkt auf reflexsympathische Dystrophie und Kausalgie), Neoplasie, vaskulitischer/angiopathischer Neuropathie und idiopathischer Neuropathie.

19. Verwendung oder das Neublastin-Polypeptid zur Verwendung gemäß einem der vorhergehenden Ansprüche, wobei das Neublastin-Polypeptid mit einer funktionellen Derivatgruppe modifiziert ist, um eine verlängerte Verweilzeit oder eine erhöhte Konzentration in Körperflüssigkeiten aufzuweisen.

20. Verwendung oder das Neublastin-Polypeptid zur Verwendung wie in Anspruch 19, wobei besagte funktionelle Derivatgruppe ausgewählt ist aus der Gruppe bestehend aus einer Polyethylenglykolgruppe, aliphatischen Estern, Amiden, N-Acylderivaten und O-Acylderivaten.

21. Verwendung oder das Neublastin-Polypeptid zur Verwendung gemäß einem der vorhergehenden Ansprüche, wobei die Aminosäuresequenz des Neublastin-Polypeptids ein Polypeptid umfasst, ausgewählt aus der Gruppe bestehend aus:
a. mindestens einem Polypeptid umfassend AA-₈₀-AA₁₄₁ von SEQ ID NO:2, AA₋₄₁-AA₁₄₀ von SEQ ID NO:2, AA₁-AA₁₄₀ aus SEQ ID NO:2, AA₂₅-AA₁₄₀ aus SEQ ID NO:2, AA₂₈-AA₁₄₀ aus SEQ ID NO:2, AA₋₈₀-AA₁₄₄ aus SEQ ID NO:4, AA₁-AA₁₄₄ aus SEQ ID NO:4, AA₁-AA₂₂₄ aus SEQ ID NO:5 oder AA₈₁-AA₂₂₄ aus SEQ ID NO:5;
b. mindestens einem Polypeptid umfassend die C-terminale Sequenz, beschrieben entweder durch AA₁₀₇-AA₁₄₀ aus SEQ ID NO:2 oder durch AA₇₆-AA₁₄₀ aus SEQ ID NO:2, welche die sieben Cys-Rückstände aufweist, die für die GDNF-Familie und die TGF-β Superfamilie charakteristisch sind;
c. mindestens einem Polypeptid umfassend SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 oder SEQ ID NO:27; und
d. mindestens einer Polypeptidsequenz, welche mehr als 70%, vorzugsweise mehr als 85%, noch weiter bevorzugt mehr als 90% und noch weiter bevorzugt mehr als 95% Aminosäurehomologie zu einer der oben in (a) - (c) genannten Sequenzen aufweist.

22. Verwendung oder das Neublastin-Polypeptid zur Verwendung gemäß einer der vorhergehenden Ansprüche, wobei die Aminosäureaequenz des Neublastin-Polypeptids ein Polypeptid umfasst, ausgewählt aus der Gruppe bestehend aus mindestens einem Polypetid, umfassend AA₄₂-AA₁₄₀ aus SEQ ID NO:2 oder AA₃₇-AA₁₄₀ aus SEQ ID NO:2.

23. Verwendung oder das Neublastin-Polypeptid zur Verwendung gemäß einer der vorhergehenden Ansprüche, wobei die pharmazeutische Zusammensetzung ferner eine Analgesie-induzierende Komponente umfasst, ausgewählt aus der Gruppe bestehend aus Opioiden, Antiarrhythmika, topischen Anageltika, lokalen Anästhetika, Antikonvulsiva, Antidepressiva, Kortikosteroiden und NSAR.

24. Verwendung oder das Neublastin-Polypeptid zur Verwendung wie in Anspruch 23, wobei die Analgesie-induzierende Komponente ein Antikonvulsivum ist.

25. Verwendung oder das Neublastin-Polypeptid zur Verwendung wie in Anspruch 23, wobei die Analgesie-induzierende Komponente Gabapentin (1-(aminomethyl)cyclohexan-Essigsäure) oder Pregabalin (S-(+)-4-amino-3-(2-methylpropyl) Buttersäure) ist.

26. Verwendung oder das Neublastin-Polypeptid zur Verwendung gemäß einem der vorhergehenden Ansprüche, wobei die Dosierung des Neublastin-Polypeptids zwischen 1 µg/kg und 30.000 µg/kg Körpergewicht des Subjektspro Dosis, zwischen 10 µg/kg und 10.000 µg/kg Körpergewicht des Subjekts pro Dosis oder zwischen 25 µg/kg und 3.000 µg/kg Körpergewicht des Subjekts pro Dosis liegt.

27. Verwendung oder das Neublastin-Polypeptid Verwendung gemäß einem der vorhergehenden Ansprüche, wobei die pharmazeutische Zusammensetzung eine zeitgesteuerte Zusammensetzung ist.

## Revendications

1. Utilisation d'un polypeptide Neublastin pour la préparation d'une composition pharmaceutique pour le traitement, la prévention ou le retardement d'une douleur neuropathique, dans laquelle la douleur neuropathique est l'allodynie, une douleur hyperalgésique ou une douleur fantôme.

2. Polypeptide Neublastin pour utilisation dans le traitement, la prévention ou le retardement d'une douleur neuropathique, dans laquelle la douleur neuropathique est l'allodynie, une douleur hyperalgésique ou une douleur fantôme.

3. Utilisation selon la revendication 1 ou le polypeptide Neublastin pour utilisation selon la revendication 2, dans laquelle la composition pharmaceutique ou le polypeptide Neublastin est préparé(e) pour administration par livraison systémique.

4. Utilisation selon la revendication 1 ou le polypeptide Neublastin pour utilisation selon la revendication 2, dans laquelle la composition pharmaceutique ou le polypeptide Neublastin est préparé(e) pour administration par livraison intraveineuse.

5. Utilisation selon la revendication 1 ou le polypeptide Neublastin pour utilisation selon la revendication 2, dans laquelle la composition pharmaceutique ou le polypeptide Neublastin est préparé(e) pour administration par livraison sous-cutanée.

6. Utilisation selon la revendication 1 ou le polypeptide Neublastin pour utilisation selon la revendication 2, dans laquellela douleur neuropathique est l'allodynie.

7. Utilisation selon la revendication 1 ou le polypeptide Neublastin pour utilisation selon la revendication 2, dans laquelle l'allodynie est l'allodynie tactile.

8. Utilisation selon la revendication 1 ou le polypeptide Neublastin pour utilisation selon la revendication 2, dans laquelle la douleur neuropathique est une douleur hyperalgésique.

9. Utilisation ou le polypeptide Neublastin pour utilisation selon la revendication 8, dans laquelle la douleur hyperalgésique est une douleur hyperalgésique thermique.

10. Utilisation selon la revendication 1 ou le polypeptide Neublastin pour utilisation selon la revendication 2, dans laquelle la douleur neuropathique est associée à une neuralgie post-herpétique, une neuropathie diabétique ou une sciatique.

11. Utilisation selon la revendication 1 ou le polypeptide Neublastin pour utilisation selon la revendication 2, dans laquelle ladite douleur neuropathique est associée à une infection par un virus.

12. Utilisation ou le polypeptide Neublastin pour utilisation selon la revendicatoin 11, dans laquelle ledit virus est sélectionné dans le groupe constitué d'un virus de l'herpès, un virus d'immunodéficience humaine (VIH), et un virus du papilloma.

13. Utilisation selon la revendication 1 ou le polypeptide Neublastin pour utilisation selon la revendication 2, dans laquelle ladite douleur neuropathique est une douleur neuropathique associée à l'administration d'un agent thérapeutique.

14. Utilisation ou le polypeptide Neublastin pour utilisation selon la revendication 13, dans laquelle ledit agent thérapeutique est un agent anti-cancer.

15. Utilisation ou le polypeptide Neublastin pour utilisation selon la revendication 14, dans laquelle ledit agent anti-cancer est sélectionné dans le groupe constitué de taxol, taxotère, cisplastine, nocodazole, vincristine, vindésine et vinblastine.

16. Utilisation ou le polypeptide Neublastin pour utilisation selon la revendication 13, dans laquelle ledit agent thérapeutique est un agent antiviral.

17. Utilisation ou le polypeptide Neublastin pour utilisation selon la revendication 16, dans laquelle ledit agent antiviral est sélectionné dans le groupe constitué de ddl, DDC, d4T, foscarnet, dapsone, métronidazole, et isoniazide.

18. Utilisation selon la revendication 1 ou le polypeptide Neublastin pour utilisation selon la revendication 2, dans laquelle la douleur neuropathique est associée à la neuropathie héréditaire (y compris, sans pour autant y être limité, l'ataxie de Friedreich, la polyneuropathie amyloïde familiale, la maladie de Tangier, la maladie de Fabry), les troubles métaboliques (y compris, sans pour autant y être limité, l'insuffisance rénale et l'hypothyroïdisme), les déficiences en vitamine (y compris, sans pour autant y être limité, la déficience en vitamine B12, la déficience en vitamine B6, et la déficience en vitamine E), les neuropathies toxiques et iatrogéniques (y compris, sans pour autant y être limité, l'alcoolisme, l'intoxication par la vitamine B6, l'intoxication par l'hexacarbone, l'amiodarone, le chloramphénicol, le disulfirame, l'isoniazide, l'or, le lithium, le métronidazole, le misonidazole, la nitrofurantoïne), les neuropathies infectieuses (y compris, sans pour autant y être limité, la lèpre et la maladie de Lyme), les neuropathies auto-immunitaires (y compris, sans pour autant y être limité, le syndrome de Guillain-Barré, la polyneuropathie chronique inflammatoire démyélinisante, la gammopathie monoclonale d'importance indéterminée, et la polyneuropathie), la névralgie trigéminale, les syndromes canalaires (y compris, sans pour autant y être limité, le tunnel de Carpel), la névralgie post-traumatique, la douleur fantôme des membres, la douleur de sclérose multiple, les syndromes de douleur régionale complexe (y compris, sans pour autant y être limité, la dystrophie sympathique réflexe et la causalgie), la néoplasie, la neuropathie vasculitique/angiopathique, et la neuropathie idiopathique.

19. Utilisation ou le polypeptide Neublastin pour utilisation selon l'une quelconque des revendications précédentes, dans laquelle le polypeptide Neublastin est modifié avec un groupe dérivé de manière à avoir un temps de séjour prolongé ou une concentration accrue dans les fluides de l'organisme.

20. Utilisation ou le polypeptide Neublastin pour utilisation selon la revendication 19, dans laquelle ledit groupe dérivé est sélectionnée dans le groupe constitué d'une groupe de polyéthylène glycol, d'esters aliphatiques, d'amides, de dérivés N-acyl et de dérivés O-acyl.

21. Utilisation ou le polypeptide Neublastin pour utilisation selon l'une quelconque des revendications précédentes, dans laquelle la séquence d'acides aminés dudit polypeptide Neublastin comprend un polypeptide sélectionné dans le groupe constitué de :
a. au moins un polypeptide comprenant AA₋₈₀-AA₁₄₀ de SEQ ID NO:2, AA-₄₁-AA₁₄₀ de SEQ ID NO:2, AA₁-AA₁₄₀ de SEQ ID NO:2, AA₂₅-AA₁₄₀ de SEQ ID NO:2, AA₂₈-AA₁₄₀ de SEQ ID NO:2, AA₋₈₀-AA₁₄₄ de SEQ ID NO:4, AA₁-AA₁₄₄ de SEQ ID NO:4, AA₁-AA₂₂₄ de SEQ ID NO:5, ou AA₈₁-AA₂₂₄ de SEQ ID NO:5;
b. au moins un polypeptide comprenant la séquence terminale C définie dans AA₁₀₇-AA₁₄₀ de SEQ ID NO:2 ou dans AA₇₆-AA₁₄₀ de SEQ ID NO:2, et qui retient les sept résidus Cys caractéristiques de la famille GDNF et de la superfamille TGF-β;
c. au moins un polypeptide comprenant SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26 ou SEQ ID NO:27; et
d. au moins une séquence de polypeptides qui présente une homologie d'acides aminés supérieure à 70%, plus préférablement 85%, encore plus préférablement 90% et encore plus préférablement 95% par rapport à l'une quelconque des séquences dans (a) - (c) ci-dessus.

22. Utilisation ou le polypeptide Neublastin pour utilisation selon l'une quelconque des revendications précédentes, dans laquelle la séquence d'acides aminés dudit polypeptide Neublastin comprend un polypeptide sélectionné dans le groupe constitué d'au moins un polypeptide comprenant AA₄₂-AA₁₄₀ de SEQ ID NO:2 ou AA₃₇-AA₁₄₀ de SEQ ID NO:2.

23. Utilisation ou le polypeptide Neublastin pour utilisation selon l'une quelconque des revendications précédentes, dans laquelle la composition pharmaceutique comprend en outre un composé induisant un effet analgésique sélectionné dans le groupe constitué d'opioïdes, anti-arrhythmiques, analgésiques topiques, anesthésiques locaux, anticonvulsants, antidépresseurs, corticostéroïdes, et AINS.

24. Utilisation ou le polypeptide Neublastin pour utilisation selon la revendication 23, dans laquellele composé induisant un effet analgésique est un anticonvulsant.

25. Utilisation ou le polypeptide Neublastin pour utilisation selon la revendication 23, dans laquelle le composé induisant un effet analgésique est la gabapentine (acide acétique 1-(aminométhyl)cyclohexane) ou la prégabaline (acide butanoïque S-(+)-4-amino-3-(2-méthylpropyl)).

26. Utilisation ou le polypeptide Neublastin pour utilisation selon l'une quelconque des revendications précédentes, dans laquelle le dosage du polypeptide Neublastin est entre 1 µg/kg et 30000 µg/kg de poids corporel du sujet, par dose, entre 10 µg/kg et 10000 µg/kg de poids corporel du sujet, par dose, ou entre 25 µg/kg et 3000 µg/kg de poids corporel du sujet, par dose.

27. Utilisation ou le polypeptide Neublastin pour utilisation selon l'une quelconque des revendications précédentes, dans laquelle la composition pharmaceutique est une composition retard.


### SEQUENCE LISTING

<110> Biogen, Inc.
   Sah, Dinah Wen-lee
<120> Treatment Using Neublastin Polypeptides
<130> 00689-507 (A118) utility
<140> Filed Herewith
   <141> 2002-02-28
<150> USSN 06/287,554
   <151> 2001-03-28
<160> 27
<170> PatentIn Ver. 2.1
<210> 1
   <211> 861
   <212> DNA
   <213> Homo sapiens
<220>
   <221> CDS
   <222> (58)..(717)
<220>
   <221> 5'UTR
   <222> (1)..(57)
<220>
   <221> 3'UTR
   <222> (718)..(861)
<220>
   <221> sig_peptide
   <222> (58)..(174)
<220>
   <221> mat_peptide
   <222> (298)..(717)
<220>
   <221> mat_peptide
   <222> (370)..(717)
<220>
   <221> mat_peptide
   <222> (379)..(717)
<220>
   <221> misc_structure
   <222> (661)..(663)
   <223> CARBOHYD: glycosylated asparagine at Asn122
<220>
   <221> misc_structure
   <222> (424)..(621)
   <223> DISULFID: Gly43-Gly108 disulfide bridge
<220>
   <221> misc_structure
   <222> (505)..(705)
   <223> DISULFID: Gly70-Gly136 disulfide bridge
<220>
   <221> misc_structure
   <222> (517)..(711)
   <223> DISULFID: Gly74-Gly138 disulfide bridge
<220>
   <221> misc_structure
   <222> (616)..(618)
   <223> DISULFID: Gly107-Gly107 interchain disulfide bridge
<400> 1
   aggagggtgg gggaacagct caacaatggc tgatgggcgc tcctggtgtt gatagag    57
   atg gaa ctt gga ctt gga ggc ctc tcc acg ctg tcc cac tgc ccc tgg   105
   Met Glu Leu Gly Leu Gly Gly Leu Ser Thr Leu Ser His Cys Pro Trp
   -80                 -75                 -70                 -65
   cct agg cgg cag cct gcc ctg tgg ccc acc ctg gcc gct ctg gct ctg   153
   Pro Arg Arg Gln Pro Ala Leu Trp Pro Thr Leu Ala Ala Leu Ala Leu
                   -60                 -55                 -50
   ctg agc agc gtc gca gag gcc tcc ctg ggc tcc gcg ccc cgc agc cct   201
   Leu Ser Ser Val Ala Glu Ala Ser Leu Gly Ser Ala Pro Arg Ser Pro
               -45                 -40                 -35
   gcc ccc cgc gaa ggc ccc ccg cct gtc ctg gcg tcc ccc gcc ggc cac   249
   Ala Pro Arg Glu Gly Pro Pro Pro Val Leu Ala Ser Pro Ala Gly His
           -30                 -25                 -20
   ctg ccg ggg gga cgc acg gcc cgc tgg tgc agt gga aga gcc cgg cgg   297
   Leu Pro Gly Gly Arg Thr Ala Arg Trp Cys Ser Gly Arg Ala Arg Arg
       -15                 -10                 -5                  -1
   ccg ccg ccg cag cct tct cgg ccc gcg ccc ccg ccg cct gca ccc cca   345
   Pro Pro Pro Gln Pro Ser Arg Pro Ala Pro Pro Pro Pro Ala Pro Pro
    1               5                  10                  15
   tct gct ctt ccc cgc ggg ggc cgc gcg gcg cgg gct ggg ggc ccg ggc   393
   Ser Ala Leu Pro Arg Gly Gly Arg Ala Ala Arg Ala Gly Gly Pro Gly
               20                  25                  30
   agc cgc gct cgg gca gcg ggg gcg cgg ggc tgc cgc ctg cgc tcg cag   441
   Ser Arg Ala Arg Ala Ala Gly Ala Arg Gly Cys Arg Leu Arg Ser Gln
           35                  40                  45
   ctg gtg ccg gtg cgc gcg ctc ggc ctg ggc cac cgc tcc gac gag ctg   489
   Leu Val Pro Val Arg Ala Leu Gly Leu Gly His Arg Ser Asp Glu Leu
       50                  55                  60
   gtg cgt ttc cgc ttc tgc agc ggc tcc tgc cgc cgc gcg cgc tct cca   537
   Val Arg Phe Arg Phe Cys Ser Gly Ser Cys Arg Arg Ala Arg Ser Pro
    65                 70                  75                  80
   cac gac ctc agc ctg gcc agc cta ctg ggc gcc ggg gcc ctg cga ccg   585
   His Asp Leu Ser Leu Ala Ser Leu Leu Gly Ala Gly Ala Leu Arg Pro
                   85                  90                  95
   ccc ccg ggc tcc cgg ccc gtc agc cag ccc tgc tgc cga ccc acg cgc   633
   Pro Pro Gly Ser Arg Pro Val Ser Gln Pro Cys Cys Arg Pro Thr Arg
               100                     105                     110
   tac gaa gcg gtc tcc ttc atg gac gtc aac agc acc tgg aga acc gtg    681
   Tyr Glu Ala Val Ser Phe Met Asp Val Asn Ser Thr Trp Arg Thr Val
           115                     120                     125
   gac cgc ctc tcc gcc acc gcc tgc ggc tgc ctg ggc tgagggctcg         727
   Asp Arg Leu Ser Ala Thr Ala Cys Gly Cys Leu Gly
       130                     135                     140
   ctccagggct ttgcagactg gacccttacc ggtggctctt cctgcctggg accctcccgc 787
   agagtcccac tagccagcgg cctcagccag ggacgaaggc ctcaaagctg agaggcccct 847
   accggtgggt gatg                                                    861
<210> 2
   <211> 220
   <212> PRT
   <213> Homo sapiens
<400> 2
   Met Glu Leu Gly Leu Gly Gly Leu Ser Thr Leu Ser His Cys Pro Trp
   -80                 -75                 -70                 -65
   Pro Arg Arg Gln Pro Ala Leu Trp Pro Thr Leu Ala Ala Leu Ala Leu
                   -60                 -55                 -50
   Leu Ser Ser Val Ala Glu Ala Ser Leu Gly Ser Ala Pro Arg Ser Pro
               -45                 -40                 -35
   Ala Pro Arg Glu Gly Pro Pro Pro Val Leu Ala Ser Pro Ala Gly His
           -30                 -25                 -20
   Leu Pro Gly Gly Arg Thr Ala Arg Trp Cys Ser Gly Arg Ala Arg Arg
       -15                 -10                 -5                  -1
   Pro Pro Pro Gln Pro Ser Arg Pro Ala Pro Pro Pro Pro Ala Pro Pro
   1               5                   10                  15
   Ser Ala Leu Pro Arg Gly Gly Arg Ala Ala Arg Ala Gly Gly Pro Gly
               20                  25                  30
   Ser Arg Ala Arg Ala Ala Gly Ala Arg Gly Cys Arg Leu Arg Ser Gln
           35                  40                  45
   Leu Val Pro Val Arg Ala Leu Gly Leu Gly His Arg Ser Asp Glu Leu
       50                  55                  60
   Val Arg Phe Arg Phe Cys Ser Gly Ser Cys Arg Arg Ala Arg Ser Pro
   65                  70                  75                  80
   His Asp Leu Ser Leu Ala Ser Leu Leu Gly Ala Gly Ala Leu Arg Pro
                   85                  90                  95
   Pro Pro Gly Ser Arg Pro Val Ser Gln Pro Cys Cys Arg Pro Thr Arg
               100                 105                 110
   Tyr Glu Ala Val Ser Phe Met Asp Val Asn Ser Thr Trp Arg Thr Val
           115                 120                 125
   Asp Arg Leu Ser Ala Thr Ala Cys Gly Cys Leu Gly
       130                 135                 140
<210> 3
   <211> 2136
   <212> DNA
   <213> Murinae gen. sp.
<220>
   <221> CDS
   <222> (975)..(1646)
<220>
   <221> 5'UTR
   <222> (1)..(974)
<220>
   <221> 3'UTR
   <222> (1647)..(2136)
<220>
   <221> sig_peptide
   <222> (975)..(1091)
<220>
   <221> mat_peptide
   <222> (1215)..(1646)
<220>
   <221> mat_peptide
   <222> (1290)..(1646)
<220>
   <221> mat_peptide
   <222> (1299)..(1646)
<400> 3
   gcggccgcga attcggcacg agggcgtctc gctgcagccc gcgatctcta ctctgcctcc 60
   tggggtcttc tccaaatgtc tagcccccac ctagagggac ctagcctagc cagcggggac 120
   cggatccgga gggtggagcg gccaggtgag ccctgaaagg tggggcgggg cgggggcgct 180
   ctgggcccca ccccgggatc tggtgacgcc ggggctggaa tttgacaccg gacggcggcg 240
   ggcaggaggc tgctgaggga tggagttggg ctcggccccc agatgcggcc cgcgggctct 300
   gccagcaaca agtccctcgg gccccagccc tcgctgcgac tggggcttgg agccctgcac 360
   ccaagggcac agaccggctg ccaaggcccc acttttaact aaaagaggcg ctgccaggtg 420
   cacaactctg ggcatgatcc acttgagctt cgggggaaag cccagcactg gtcccaggag 480
   aggcgcctag aaggacacgg accaggaccc ctttggtatg gagtgaacgc tgagcatgga 540
   gtggaaggaa ctcaagttac tactttctcc aaccaccctg gtaccttcag ccctgaagta 600
   cagagcagaa gggtcttaga agacaggacc acagctgtgt gagtctcccc cctgaggcct 660
   tagacgatct ctgagctcag ctgagctttg tttgcccatc tggagaagtg agccattgat 720
   tgaccttgtg gcatcgcgaa ggaacaggtc ctgccaagca cctaacacag agagcaaggt 780
   tctccatcgc agctaccgct gctgagttga ctctagctac tccaacctcc tgggtcgctt 840
   cgagagactg gagtggaagg aggaataccc caaaggataa ctaactcatc tttcagtttg 900
   caagctgccg caggaagagg gtggggaaac gggtccacga aggcttctga tgggagcttc 960
   tggagccgaa agct atg gaa ctg gga ctt gca gag cct act gca ttg tcc     1010
                   Met Glu Leu Gly Leu Ala Glu Pro Thr Ala Leu Ser
                   -80                 -75                 -70
   cac tgc ctc cgg cct agg tgg cag tca gcc tgg tgg cca acc cta gct     1058
   His Cys Leu Arg Pro Arg Trp Gln Ser Ala Trp Trp Pro Thr Leu Ala
               -65                 -60                 -55
   gtt cta gcc ctg ctg agc tgc gtc aca gaa gct tcc ctg gac cca atg     1106
   Val Leu Ala Leu Leu Ser Cys Val Thr Glu Ala Ser Leu Asp Pro Met
           -50                 -45                 -40
   tcc cgc agc ccc gcc gct cgc gac ggt ccc tca ccg gtc ttg gcg ccc     1154
   Ser Arg Ser Pro Ala Ala Arg Asp Gly Pro Ser Pro Val Leu Ala Pro
       -35                 -30                 -25
   ccc acg gac cac ctg cct ggg gga cac act gcg cat ttg tgc agc gaa     1202
   Pro Thr Asp His Leu Pro Gly Gly His Thr Ala His Leu Cys Ser Glu
   -20                 -15                 -10                  -5
   aga acc ctg cga ccc ccg cct cag tct cct cag ccc gca ccc ccg ccg     1250
   Arg Thr Leu Arg Pro Pro Pro Gln Ser Pro Gln Pro Ala Pro Pro Pro
                -1   1              5                  10
   cct ggt ccc gcg ctc cag tct cct ccc gct gcg ctc cgc ggg gca cgc     1298
   Pro Gly Pro Ala Leu Gln Ser Pro Pro Ala Ala Leu Arg Gly Ala Arg
           15                  20                  25
   gcg gcg cgt gca gga acc cgg agc agc cgc gca cgg acc aca gat gcg     1346
   Ala Ala Arg Ala Gly Thr Arg Ser Ser Arg Ala Arg Thr Thr Asp Ala
       30                  35                  40
   cgc ggc tgc cgc ctg cgc tcg cag ctg gtg ccg gtg agc gcg ctc ggc     1394
   Arg Gly Cys Arg Leu Arg Ser Gln Leu Val Pro Val Ser Ala Leu Gly
   45                  50                  55                  60
   cta ggc cac agc tcc gac gag ctg ata cgt ttc cgc ttc tgc agc ggc     1442
   Leu Gly His Ser Ser Asp Glu Leu Ile Arg Phe Arg Phe Cys Ser Gly
                   65                  70                  75
   tcg tgc cgc cga gca cgc tcc cag cac gat ctc agt ctg gcc agc cta     1490
   Ser Cys Arg Arg Ala Arg Ser Gln His Asp Leu Ser Leu Ala Ser Leu
               80                  85                  90
   ctg ggc gct ggg gcc cta cgg tcg cct ccc ggg tcc cgg ccg atc agc     1538
   Leu Gly Ala Gly Ala Leu Arg Ser Pro Pro Gly Ser Arg Pro Ile Ser
           95                 100                 105
   cag ccc tgc tgc cgg ccc act cgc tat gag gcc gtc tcc ttc atg gac     1586
   Gln Pro Cys Cys Arg Pro Thr Arg Tyr Glu Ala Val Ser Phe Met Asp
       110                 115                 120
   gtg aac agc acc tgg agg acc gtg gac cac ctc tcc gcc act gcc tgc     1634
   Val Asn Ser Thr Trp Arg Thr Val Asp His Leu Ser Ala Thr Ala Cys
   125                 130                 135                 140
   ggc tgt ctg ggc tgaggatgat ctatctccaa gcctttgcac actagaccca         1686
   Gly Cys Leu Gly
   tgtgttgccc tacctggaac agctccaccg ggcctcacta accaggagcc tcaactcagc 1746
   aggatatgga ggctgcagag ctcaggcccc aggccggtga gtgacagacg tcgtcggcat 1806
   gacagacaga gtgaaagatg tcggaaccac tgaccaacag tcccaagttg ttcatggatc 1866
   ccagctctac agacaggaga aacctcagct aaagagaact cctctgggag aatccagaaa 1926
   tggccctctg tcctggggaa tgaattttga agagatatat atacatatat acattgtagt 1986
   cgcgttgctg gaccagcctg tgctgaaacc agtcccgtgt tcacttgtgg aagccgaagc 2046
   cctatttatt atttctaaat tatttattta ctttgaaaaa aaacggccaa gtcggcctcc 2106
   ctttagtgag ggttaatttg tgatcccggg                                  2136
<210> 4
   <211> 224
   <212> PRT
   <213> Murinae gen. sp.
<400> 4
   Met Glu Leu Gly Leu Ala Glu Pro Thr Ala Leu Ser His Cys Leu Arg
   -80                 -75                 -70                 -65
   Pro Arg Trp Gln Ser Ala Trp Trp Pro Thr Leu Ala Val Leu Ala Leu
                   -60                 -55                 -50
   Leu Ser Cys Val Thr Glu Ala Ser Leu Asp Pro Met Ser Arg Ser Pro
               -45                 -40                 -35
   Ala Ala Arg Asp Gly Pro Ser Pro Val Leu Ala Pro Pro Thr Asp His
           -30                 -25                 -20
   Leu Pro Gly Gly His Thr Ala His Leu Cys Ser Glu Arg Thr Leu Arg
       -15                 -10                 -5                  -1
   Pro Pro Pro Gln Ser Pro Gln Pro Ala Pro Pro Pro Pro Gly Pro Ala
   1               5                   10                  15
   Leu Gln Ser Pro Pro Ala Ala Leu Arg Gly Ala Arg Ala Ala Arg Ala
               20                  25                  30
   Gly Thr Arg Ser Ser Arg Ala Arg Thr Thr Asp Ala Arg Gly Cys Arg
           35                  40                  45
   Leu Arg Ser Gln Leu Val Pro Val Ser Ala Leu Gly Leu Gly His Ser
       50                  55                  60
   Ser Asp Glu Leu Ile Arg Phe Arg Phe Cys Ser Gly Ser Cys Arg Arg
   65                  70                  75                  80
   Ala Arg Ser Gln His Asp Leu Ser Leu Ala Ser Leu Leu Gly Ala Gly
                   85                  90                  95
   Ala Leu Arg Ser Pro Pro Gly Ser Arg Pro Ile Ser Gln Pro Cys Cys
               100                 105                 110
   Arg Pro Thr Arg Tyr Glu Ala Val Ser Phe Met Asp Val Asn Ser Thr
           115                 120                 125
   Trp Arg Thr Val Asp His Leu Ser Ala Thr Ala Cys Gly Cys Leu Gly
       130                 135                 140
<210> 5
   <211> 224
   <212> PRT
   <213> Rattus sp.
<220>
   <221> SIGNAL
   <222> (1)..(39)
<220>
   <221> PROPEP
   <222> (40)..(80)
<220>
   <221> PEPTIDE
   <222> (81)..(224)
<220>
   <221> PEPTIDE
   <222> (109)..(224 )
<220>
   <221> PEPTIDE
   <222> (112)..(224)
<220>
   <221> CARBOHYD
   <222> (206)
<220>
   <221> DISULFID
   <222> (127)..(192)
<220>
   <221> DISULFID
   <222> (154)..(220)
<220>
   <221> DISULFID
   <222> (158)..(222)
<220>
   <221> DISULFID
   <222> (191)
   <223> Interchain disulfide link
<400> 5
   Met Glu Leu Gly Leu Gly Glu Pro Thr Ala Leu Ser His Cys Leu Arg
   1               5                   10                  15
   Pro Arg Trp Gln Pro Ala Leu Trp Pro Thr Leu Ala Ala Leu Ala Leu
               20                  25                  30
   Leu Ser Ser Val Thr Glu Ala Ser Leu Asp Pro Met Ser Arg Ser Pro
           35                  40                  45
   Ala Ser Arg Asp Val Pro Ser Pro Val Leu Ala Pro Pro Thr Asp Tyr
       50                  55                  60
   Leu Pro Gly Gly His Thr Ala His Leu Cys Ser Glu Arg Ala Leu Arg
   65                  70                  75                  80
   Pro Pro Pro Gln Ser Pro Gln Pro Ala Pro Pro Pro Pro Gly Pro Ala
   85 90 95
   Leu Gln Ser Pro Pro Ala Ala Leu Arg Gly Ala Arg Ala Ala Arg Ala
   100 105 110
   Gly Thr Arg Ser Ser Arg Ala Arg Ala Thr Asp Ala Arg Gly Cys Arg
   115 120 125
   Leu Arg Ser Gln Leu Val Pro Val Ser Ala Leu Gly Leu Gly His Ser
   130 135 140
   Ser Asp Glu Leu Ile Arg Phe Arg Phe Cys Ser Gly Ser Cys Arg Arg
   145 150 155 160
   Ala Arg Ser Pro His Asp Leu Ser Leu Ala Ser Leu Leu Gly Ala Gly
   165 170 175
   Ala Leu Arg Ser Pro Pro Gly Ser Arg Pro Ile Ser Gln Pro Cys Cys
   180 185 190
   Arg Pro Thr Arg Tyr Glu Ala Val Ser Phe Met Asp Val Asn Ser Thr
   195 200 205
   Trp Arg Thr Val Asp His Leu Ser Ala Thr Ala Cys Gly Cys Leu Gly
   210 215 220
<210> 6
   <211> 197
   <212> PRT
   <213> Homo sapiens
<400> 6
   Met Gln Arg Trp Lys Ala Ala Ala Leu Ala Ser Val Leu Cys Ser Ser
   1 5 10 15
   Val Leu Ser Ile Trp Met Cys Arg Glu Gly Leu Leu Leu Ser His Arg
   20 25 30
   Leu Gly Pro Ala Leu Val Pro Leu His Arg Leu Pro Arg Thr Leu Asp
   35 40 45
   Ala Arg Ile Ala Arg Leu Ala Gln Tyr Arg Ala Leu Leu Gln Gly Ala
   50 55 60
   Pro Asp Ala Met Glu Leu Arg Glu Leu Thr Pro Trp Ala Gly Arg Pro
   65 70 75 80
   Pro Gly Pro Arg Arg Arg Ala Gly Pro Arg Arg Arg Arg Ala Arg Ala
   85 90 95
   Arg Leu Gly Ala Arg Pro Cys Gly Leu Arg Glu Leu Glu Val Arg Val
   100 105 110
   Ser Glu Leu Gly Leu Gly Tyr Ala Ser Asp Glu Thr Val Leu Phe Arg
   115 120 125
   Tyr Cys Ala Gly Ala Cys Glu Ala Ala Ala Arg Val Tyr Asp Leu Gly
   130 135 140
   Leu Arg Arg Leu Arg Gln Arg Arg Arg Leu Arg Arg Glu Arg Val Arg
   145                 150                 155                 160
   Ala Gln Pro Cys Cys Arg Pro Thr Ala Tyr Glu Asp Glu Val Ser Phe
                   165                 170                 175
   Leu Asp Ala His Ser Arg Tyr His Thr Val His Glu Leu Ser Ala Arg
               180                 185                 190
   Glu Cys Ala Cys Val
           195
<210> 7
   <211> 156
   <212> PRT
   <213> Homo sapiens
<400> 7
   Met Ala Val Gly Lys Phe Leu Leu Gly Ser Leu Leu Leu Leu Ser Leu
    1               5                  10                  15
   Gln Leu Gly Gln Gly Trp Gly Pro Asp Ala Arg Gly Val Pro Val Ala
               20                  25                  30
   Asp Gly Glu Phe Ser Ser Glu Gln Val Ala Lys Ala Gly Gly Thr Trp
           35                  40                  45
   Leu Gly Thr His Arg Pro Leu Ala Arg Leu Arg Arg Ala Leu Ser Gly
       50                  55                  60
   Pro Cys Gln Leu Trp Ser Leu Thr Leu Ser Val Ala Glu Leu Gly Leu
   65                  70                  75                  80
   Gly Tyr Ala Ser Glu Glu Lys Val Ile Phe Arg Tyr Cys Ala Gly Ser
                   85                  90                  95
   Cys Pro Arg Gly Ala Arg Thr Gln His Gly Leu Ala Leu Ala Arg Leu
               100                 105                 110
   Gln Gly Gln Gly Arg Ala His Gly Gly Pro Cys Cys Arg Pro Thr Arg
           115                 120                 125
   Tyr Thr Asp Val Ala Phe Leu Asp Asp Arg His Arg Trp Gln Arg Leu
       130                 135                 140
   Pro Gln Leu Ser Ala Ala Ala Cys Gly Cys Gly Gly
   145                 150                 155
<210> 8
   <211> 211
   <212> PRT
   <213> Homo sapiens
<400> 8
   Met Lys Leu Trp Asp Val Val Ala Val Cys Leu Val Leu Leu His Thr
   1               5                   10                  15
   Ala Ser Ala Phe Pro Leu Pro Ala Gly Lys Arg Pro Pro Glu Ala Pro
               20                  25                  30
   Ala Glu Asp Arg Ser Leu Gly Arg Arg Arg Ala Pro Phe Ala Leu Ser
           35                  40                  45
   Ser Asp Ser Asn Met Pro Glu Asp Tyr Pro Asp Gln Phe Asp Asp Val
       50                  55                  60
   Met Asp Phe Ile Gln Ala Thr Ile Lys Arg Leu Lys Arg Ser Pro Asp
   65                  70                  75                  80
   Lys Gln Met Ala Val Leu Pro Arg Arg Glu Arg Asn Arg Gln Ala Ala
                   85                  90                  95
   Ala Ala Asn Pro Glu Asn Ser Arg Gly Lys Gly Arg Arg Gly Gln Arg
               100                 105                 110
   Gly Lys Asn Arg Gly Cys Val Leu Thr Ala Ile His Leu Asn Val Thr
           115                 120                 125
   Asp Leu Gly Leu Gly Tyr Glu Thr Lys Glu Glu Leu Ile Phe Arg Tyr
       130                 135                 140
   Cys Ser Gly Ser Cys Asp Ala Ala Glu Thr Thr Tyr Asp Lys Ile Leu
   145                 150                 155                 160
   Lys Asn Leu Ser Arg Asn Arg Arg Leu Val Ser Asp Lys Val Gly Gln
                   165                 170                 175
   Ala Cys Cys Arg Pro Ile Ala Phe Asp Asp Asp Leu Ser Phe Leu Asp
               180                 185                 190
   Asp Asn Leu Val Tyr His Ile Leu Arg Lys His Ser Ala Lys Arg Cys
           195                 200                 205
   Gly Cys Ile
       210
<210> 9
   <211> 39
   <212> DNA
   <213> Homo sapiens
<400> 9
   aaggaaaaaa gcggccgcca tggaacttgg acttggagg 39
<210> 10
   <211> 39
   <212> DNA
   <213> Homo sapiens
<400> 10
   ttttttcctt ggcggccgct cagcccaggc agccgcagg 39
<210> 11
   <211> 140
   <212> PRT
   <213> Homo sapiens
<220>
   <221> CARBOHYD
   <222> (122)
   <223> glycosylated asparagine
<400> 11
   Pro Pro Pro Gln Pro Ser Arg Pro Ala Pro Pro Pro Pro Ala Pro Pro
   1               5                   10                  15
   Ser Ala Leu Pro Arg Gly Gly Arg Ala Ala Arg Ala Gly Gly Pro Gly
               20                  25                  30
   Ser Arg Ala Arg Ala Ala Gly Ala Arg Gly Cys Arg Leu Arg Ser Gln
           35                  40                  45
   Leu Val Pro Val Arg Ala Leu Gly Leu Gly His Arg Ser Asp Glu Leu
       50                  55                  60
   Val Arg Phe Arg Phe Cys Ser Gly Ser Cys Arg Arg Ala Arg Ser Pro
   65                  70                  75                  80
   His Asp Leu Ser Leu Ala Ser Leu Leu Gly Ala Gly Ala Leu Arg Pro
                   85                  90                  95
   Pro Pro Gly Ser Arg Pro Val Ser Gln Pro Cys Cys Arg Pro Thr Arg
               100                 105                 110
   Tyr Glu Ala Val Ser Phe Met Asp Val Asn Ser Thr Trp Arg Thr Val
           115                 120                 125
   Asp Arg Leu Ser Ala Thr Ala Cys Gly Cys Leu Gly
       130                 135                 140
<210> 12
   <211> 116
   <212> PRT
   <213> Homo sapiens
<220>
   <221> CARBOHYD
   <222> (98)
   <223> glycosylated asparagine
<400> 12
   Ala Ala Arg Ala Gly Gly Pro Gly Ser Arg Ala Arg Ala Ala Gly Ala
    1               5                  10                  15
   Arg Gly Cys Arg Leu Arg Ser Gln Leu Val Pro Val Arg Ala Leu Gly
               20                  25                  30
   Leu Gly His Arg Ser Asp Glu Leu Val Arg Phe Arg Phe Cys Ser Gly
           35                  40                  45
   Ser Cys Arg Arg Ala Arg Ser Pro His Asp Leu Ser Leu Ala Ser Leu
       50                  55                  60
   Leu Gly Ala Gly Ala Leu Arg Pro Pro Pro Gly Ser Arg Pro Val Ser
   65                  70                  75                  80
   Gln Pro Cys Cys Arg Pro Thr Arg Tyr Glu Ala Val Ser Phe Met Asp
                   85                  90                  95
   Val Asn Ser Thr Trp Arg Thr Val Asp Arg Leu Ser Ala Thr Ala Cys
               100                 105                 110
   Gly Cys Leu Gly
           115
<210> 13
   <211> 113
   <212> PRT
   <213> Homo sapiens
<220>
   <221> CARBOHYD
   <222> (95)
   <223> glycosylated asparagine
<400> 13
   Ala Gly Gly Pro Gly Ser Arg Ala Arg Ala Ala Gly Ala Arg Gly Cys
    1               5                  10                  15
   Arg Leu Arg Ser Gln Leu Val Pro Val Arg Ala Leu Gly Leu Gly His
               20                  25                  30
   Arg Ser Asp Glu Leu Val Arg Phe Arg Phe Cys Ser Gly Ser Cys Arg
           35                  40                  45
   Arg Ala Arg Ser Pro His Asp Leu Ser Leu Ala Ser Leu Leu Gly Ala
       50                  55                  60
   Gly Ala Leu Arg Pro Pro Pro Gly Ser Arg Pro Val Ser Gln Pro Cys
   65                  70                  75                  80
   Cys Arg Pro Thr Arg Tyr Glu Ala Val Ser Phe Met Asp Val Asn Ser
                   85                  90                  95
   Thr Trp Arg Thr Val Asp Arg Leu Ser Ala Thr Ala Cys Gly Cys Leu
               100                 105                 110
   Gly
<210> 14
   <211> 112
   <212> PRT
   <213> Homo sapiens
<400> 14
   Gly Gly Pro Gly Ser Arg Ala Arg Ala Ala Gly Ala Arg Gly Cys Arg
   1               5                   10                  15
   Leu Arg Ser Gln Leu Val Pro Val Arg Ala Leu Gly Leu Gly His Arg
               20                  25                  30
   Ser Asp Glu Leu Val Arg Phe Arg Phe Cys Ser Gly Ser Cys Arg Arg
           35                  40                  45
   Ala Arg Ser Pro His Asp Leu Ser Leu Ala Ser Leu Leu Gly Ala Gly
       50                  55                  60
   Ala Leu Arg Pro Pro Pro Gly Ser Arg Pro Val Ser Gln Pro Cys Cys
   65                  70                  75                  80
   Arg Pro Thr Arg Tyr Glu Ala Val Ser Phe Met Asp Val Asn Ser Thr
                   85                  90                  95
   Trp Arg Thr Val Asp Arg Leu Ser Ala Thr Ala Cys Gly Cys Leu Gly
               100                 105                 110
<210> 15
   <211> 111
   <212> PRT
   <213> Homo sapiens
<400> 15
   Gly Pro Gly Ser Arg Ala Arg Ala Ala Gly Ala Arg Gly Cys Arg Leu
   1               5                   10                  15
   Arg Ser Gln Leu Val Pro Val Arg Ala Leu Gly Leu Gly His Arg Ser
               20                  25                  30
   Asp Glu Leu Val Arg Phe Arg Phe Cys Ser Gly Ser Cys Arg Arg Ala
           35                  40                  45
   Arg Ser Pro His Asp Leu Ser Leu Ala Ser Leu Leu Gly Ala Gly Ala
       50                  55                  60
   Leu Arg Pro Pro Pro Gly Ser Arg Pro Val Ser Gln Pro Cys Cys Arg
   65                  70                  75                  80
   Pro Thr Arg Tyr Glu Ala Val Ser Phe Met Asp Val Asn Ser Thr Trp
                   85                  90                  95
   Arg Thr Val Asp Arg Leu Ser Ala Thr Ala Cys Gly Cys Leu Gly
               100                 105                 110
<210> 16
   <211> 110
   <212> PRT
   <213> Homo sapiens
<400> 16
   Pro Gly Ser Arg Ala Arg Ala Ala Gly Ala Arg Gly Cys Arg Leu Arg
   1               5                   10                  15
   Ser Gln Leu Val Pro Val Arg Ala Leu Gly Leu Gly His Arg Ser Asp
               20                  25                  30
   Glu Leu Val Arg Phe Arg Phe Cys Ser Gly Ser Cys Arg Arg Ala Arg
           35                  40                  45
   Ser Pro His Asp Leu Ser Leu Ala Ser Leu Leu Gly Ala Gly Ala Leu
       50                  55                  60
   Arg Pro Pro Pro Gly Ser Arg Pro Val Ser Gln Pro Cys Cys Arg Pro
   65                  70                  75                  80
   Thr Arg Tyr Glu Ala Val Ser Phe Met Asp Val Asn Ser Thr Trp Arg
                   85                  90                  95
   Thr Val Asp Arg Leu Ser Ala Thr Ala Cys Gly Cys Leu Gly
               100                 105                 110
<210> 17
   <211> 109
   <212> PRT
   <213> Homo sapiens
<400> 17
   Gly Ser Arg Ala Arg Ala Ala Gly Ala Arg Gly Cys Arg Leu Arg Ser
   1               5                   10                  15
   Gln Leu Val Pro Val Arg Ala Leu Gly Leu Gly His Arg Ser Asp Glu
               20                  25                  30
   Leu Val Arg Phe Arg Phe Cys Ser Gly Ser Cys Arg Arg Ala Arg Ser
           35                  40                  45
   Pro His Asp Leu Ser Leu Ala Ser Leu Leu Gly Ala Gly Ala Leu Arg
       50                  55                  60
   Pro Pro Pro Gly Ser Arg Pro Val Ser Gln Pro Cys Cys Arg Pro Thr
   65                  70                  75                  80
   Arg Tyr Glu Ala Val Ser Phe Met Asp Val Asn Ser Thr Trp Arg Thr
                   85                  90                  95
   Val Asp Arg Leu Ser Ala Thr Ala Cys Gly Cys Leu Gly
               100                 105
<210> 18
   <211> 108
   <212> PRT
   <213> Homo sapiens
<400> 18
   Ser Arg Ala Arg Ala Ala Gly Ala Arg Gly Cys Arg Leu Arg Ser Gln
   1 5 10 15
   Leu Val Pro Val Arg Ala Leu Gly Leu Gly His Arg Ser Asp Glu Leu
   20 25 30
   Val Arg Phe Arg Phe Cys Ser Gly Ser Cys Arg Arg Ala Arg Ser Pro
   35 40 45
   His Asp Leu Ser Leu Ala Ser Leu Leu Gly Ala Gly Ala Leu Arg Pro
   50 55 60
   Pro Pro Gly Ser Arg Pro Val Ser Gln Pro Cys Cys Arg Pro Thr Arg
   65 70 75 80
   Tyr Glu Ala Val Ser Phe Met Asp Val Asn Ser Thr Trp Arg Thr Val
   85 90 95
   Asp Arg Leu Ser Ala Thr Ala Cys Gly Cys Leu Gly
   100 105
<210> 19
   <211> 107
   <212> PRT
   <213> Homo sapiens
<400> 19
   Arg Ala Arg Ala Ala Gly Ala Arg Gly Cys Arg Leu Arg Ser Gln Leu
   1 5 10 15
   Val Pro Val Arg Ala Leu Gly Leu Gly His Arg Ser Asp Glu Leu Val
   20 25 30
   Arg Phe Arg Phe Cys Ser Gly Ser Cys Arg Arg Ala Arg Ser Pro His
   35 40 45
   Asp Leu Ser Leu Ala Ser Leu Leu Gly Ala Gly Ala Leu Arg Pro Pro
   50 55 60
   Pro Gly Ser Arg Pro Val Ser Gln Pro Cys Cys Arg Pro Thr Arg Tyr
   65 70 75 80
   Glu Ala Val Ser Phe Met Asp Val Asn Ser Thr Trp Arg Thr Val Asp
   85 90 95
   Arg Leu Ser Ala Thr Ala Cys Gly Cys Leu Gly
   100 105
<210> 20
   <211> 106
   <212> PRT
   <213> Homo sapiens
<400> 20
   Ala Arg Ala Ala Gly Ala Arg Gly Cys Arg Leu Arg Ser Gln Leu Val
   1               5                   10                  15
   Pro Val Arg Ala Leu Gly Leu Gly His Arg Ser Asp Glu Leu Val Arg
               20                  25                  30
   Phe Arg Phe Cys Ser Gly Ser Cys Arg Arg Ala Arg Ser Pro His Asp
           35                  40                  45
   Leu Ser Leu Ala Ser Leu Leu Gly Ala Gly Ala Leu Arg Pro Pro Pro
       50                  55                  60
   Gly Ser Arg Pro Val Ser Gln Pro Cys Cys Arg Pro Thr Arg Tyr Glu
   65                  70                  75                  80
   Ala Val Ser Phe Met Asp Val Asn Ser Thr Trp Arg Thr Val Asp Arg
                   85                  90                  95
   Leu Ser Ala Thr Ala Cys Gly Cys Leu Gly
               100                 105
<210> 21
   <211> 105
   <212> PRT
   <213> Homo sapiens
<400> 21
   Arg Ala Ala Gly Ala Arg Gly Cys Arg Leu Arg Ser Gln Leu Val Pro
   1               5                   10                  15
   Val Arg Ala Leu Gly Leu Gly His Arg Ser Asp Glu Leu Val Arg Phe
               20                  25                  30
   Arg Phe Cys Ser Gly Ser Cys Arg Arg Ala Arg Ser Pro His Asp Leu
           35                  40                  45
   Ser Leu Ala Ser Leu Leu Gly Ala Gly Ala Leu Arg Pro Pro Pro Gly
       50                  55                  60
   Ser Arg Pro Val Ser Gln Pro Cys Cys Arg Pro Thr Arg Tyr Glu Ala
   65                  70                  75                  80
   Val Ser Phe Met Asp Val Asn Ser Thr Trp Arg Thr Val Asp Arg Leu
                   85                  90                  95
   Ser Ala Thr Ala Cys Gly Cys Leu Gly
               100                 105
<210> 22
   <211> 104
   <212> PRT
   <213> Homo sapiens
<400> 22
   Ala Ala Gly Ala Arg Gly Cys Arg Leu Arg Ser Gln Leu Val Pro Val
   1               5                   10                  15
   Arg Ala Leu Gly Leu Gly His Arg Ser Asp Glu Leu Val Arg Phe Arg
               20                  25                  30
   Phe Cys Ser Gly Ser Cys Arg Arg Ala Arg Ser Pro His Asp Leu Ser
           35                  40                  45
   Leu Ala Ser Leu Leu Gly Ala Gly Ala Leu Arg Pro Pro Pro Gly Ser
       50                  55                  60
   Arg Pro Val Ser Gln Pro Cys Cys Arg Pro Thr Arg Tyr Glu Ala Val
   65                  70                  75                  80
   Ser Phe Met Asp Val Asn Ser Thr Trp Arg Thr Val Asp Arg Leu Ser
                   85                  90                  95
   Ala Thr Ala Cys Gly Cys Leu Gly
               100
<210> 23
   <211> 103
   <212> PRT
   <213> Homo sapiens
<400> 23
   Ala Gly Ala Arg Gly Cys Arg Leu Arg Ser Gln Leu Val Pro Val Arg
   1               5                   10                  15
   Ala Leu Gly Leu Gly His Arg Ser Asp Glu Leu Val Arg Phe Arg Phe
               20                  25                  30
   Cys Ser Gly Ser Cys Arg Arg Ala Arg Ser Pro His Asp Leu Ser Leu
           35                  40                  45
   Ala Ser Leu Leu Gly Ala Gly Ala Leu Arg Pro Pro Pro Gly Ser Arg
       50                  55                  60
   Pro Val Ser Gln Pro Cys Cys Arg Pro Thr Arg Tyr Glu Ala Val Ser
   65                  70                  75                  80
   Phe Met Asp Val Asn Ser Thr Trp Arg Thr Val Asp Arg Leu Ser Ala
                   85                  90                  95
   Thr Ala Cys Gly Cys Leu Gly
               100
<210> 24
   <211> 102
   <212> PRT
   <213> Homo sapiens
<400> 24
   Gly Ala Arg Gly Cys Arg Leu Arg Ser Gln Leu Val Pro Val Arg Ala
   1               5                   10                  15
   Leu Gly Leu Gly His Arg Ser Asp Glu Leu Val Arg Phe Arg Phe Cys
               20                  25                  30
   Ser Gly Ser Cys Arg Arg Ala Arg Ser Pro His Asp Leu Ser Leu Ala
           35                  40                  45
   Ser Leu Leu Gly Ala Gly Ala Leu Arg Pro Pro Pro Gly Ser Arg Pro
       50                  55                  60
   Val Ser Gln Pro Cys Cys Arg Pro Thr Arg Tyr Glu Ala Val Ser Phe
   65                  70                  75                  80
   Met Asp Val Asn Ser Thr Trp Arg Thr Val Asp Arg Leu Ser Ala Thr
                   85                  90                  95
   Ala Cys Gly Cys Leu Gly
               100
<210> 25
   <211> 101
   <212> PRT
   <213> Homo sapiens
<400> 25
   Ala Arg Gly Cys Arg Leu Arg Ser Gln Leu Val Pro Val Arg Ala Leu
   1               5                   10                  15
   Gly Leu Gly His Arg Ser Asp Glu Leu Val Arg Phe Arg Phe Cys Ser
               20                  25                  30
   Gly Ser Cys Arg Arg Ala Arg Ser Pro His Asp Leu Ser Leu Ala Ser
           35                  40                  45
   Leu Leu Gly Ala Gly Ala Leu Arg Pro Pro Pro Gly Ser Arg Pro Val
       50                  55                  60
   Ser Gln Pro Cys Cys Arg Pro Thr Arg Tyr Glu Ala Val Ser Phe Met
   65                  70                  75                  80
   Asp Val Asn Ser Thr Trp Arg Thr Val Asp Arg Leu Ser Ala Thr Ala
                   85                  90                  95
   Cys Gly Cys Leu Gly
               100
<210> 26
   <211> 100
   <212> PRT
   <213> Homo sapiens
<400> 26
   Arg Gly Cys Arg Leu Arg Ser Gln Leu Val Pro Val Arg Ala Leu Gly
   1               5                   10                  15
   Leu Gly His Arg Ser Asp Glu Leu Val Arg Phe Arg Phe Cys Ser Gly
                   20                  25                  30
   Ser Cys Arg Arg Ala Arg Ser Pro His Asp Leu Ser Leu Ala Ser Leu
               35                  40                  45
   Leu Gly Ala Gly Ala Leu Arg Pro Pro Pro Gly Ser Arg Pro Val Ser
       50                  55                  60
   Gln Pro Cys Cys Arg Pro Thr Arg Tyr Glu Ala Val Ser Phe Met Asp
   65                  70                  75                      80
   Val Asn Ser Thr Trp Arg Thr Val Asp Arg Leu Ser Ala Thr Ala Cys
                   85                  90                  95
   Gly Cys Leu Gly
               100
<210> 27
   <211> 99
   <212> PRT
   <213> Homo sapiens
<400> 27
   Gly Cys Arg Leu Arg Ser Gln Leu Val Pro Val Arg Ala Leu Gly Leu
   1               5                   10                  15
   Gly His Arg Ser Asp Glu Leu Val Arg Phe Arg Phe Cys Ser Gly Ser
               20                  25                  30
   Cys Arg Arg Ala Arg Ser Pro His Asp Leu Ser Leu Ala Ser Leu Leu
           35                  40                  45
   Gly Ala Gly Ala Leu Arg Pro Pro Pro Gly Ser Arg Pro Val Ser Gln
       50                  55                  60
   Pro Cys Cys Arg Pro Thr Arg Tyr Glu Ala Val Ser Phe Met Asp Val
   65                  70                  75                  80
   Asn Ser Thr Trp Arg Thr Val Asp Arg Leu Ser Ala Thr Ala Cys Gly
                   85                  90                  95
   Cys Leu Gly